# EUROPEAN PATENT APPLICATION

(11) **EP 3 960 758 A1**
(43) Date of publication of application: **02.03.2022**
(21) Application number: 20795091.6
(22) Date of filing: 15.04.2020
(51) Int. Cl.: C07K 14/47, C07K 16/32, G01N 33/574, G01N 33/68

(54) **CANCER DETECTION METHOD AND DETECTION REAGENT**

(30) Priority: 24.04.2019 JP 2019082912
(71) Applicant: Japanese Foundation For Cancer Research, Tokyo 135-8550 (JP); Osaka University, Suita-shi, Osaka 565-0871 (JP); Tosoh Corporation, Shunan-shi, Yamaguchi 746-8501 (JP)
(72) Inventor: UEDA, Koji, Tokyo 135-8550 (JP); OHNISHI, Naomi, Tokyo 135-8550 (JP); TSUJIKAWA, Kazutake, Suita-shi, Osaka 565-0871 (JP); NONOMURA, Norio, Suita-shi, Osaka 565-0871 (JP); UEMURA, Motohide, Suita-shi, Osaka 565-0871 (JP); JINGUSHI, Kentaro, Suita-shi, Osaka 565-0871 (JP); OHTAKE, Norihisa, Ayase-shi, Kanagawa 252-1123 (JP); KAWAI, Yasutoshi, Ayase-shi, Kanagawa 252-1123 (JP)
(74) Representative: Vigand, Philippe
(86) International application number: PCT/JP2020/016607
(87) International publication number: WO 2020/218121

(57) **Abstract**

An object of the invention is to provide a method for detecting cancer in a simple and highly accurate manner, and a reagent that can be used in the method. A method for detecting cancer (excluding renal cell cancer), which comprises measuring the level of Azurocidin (AZU1) in a sample, in which it is determined that cancer is detected when a measured value exceeds a preset reference value. The cancer is preferably selected from the group consisting of stomach cancer, breast cancer, colorectal cancer, and lung cancer. A reagent containing an antibody that specifically recognizes AZU1 is used in detecting cancer (excluding renal cell cancer).

## Description

### TECHNICAL FIELD

The present invention relates to a method for detecting cancer using Azurocidin (hereinafter also referred to as "AZU1") as a measuring object and a reagent for detecting cancer using the same.

### BACKGROUND ART

Tumor markers for detecting cancer generally include those shown in Table 1. However, all of these markers show a low positive rate in the early stages of cancer, and many of the markers have problems, such as, for example, false positivity in benign tumors or inflammations and the inability to detect a highly malignant tumor. Therefore, there is a demand for discovering a tumor marker capable of detecting these types of cancer in a highly accurate manner and developing a test method using such a marker.

### [Table 1]

**(Table 1)**

| **Disease** | **Tumor marker** | **Characteristics** |
|---|---|---|
| Stomach cancer | CEA | No organ specificity, with false positivity |
| | CA19-9 | Only for late stage cancer |
| | STN | Relatively high specificity, with low sensitivity |
| Breast cancer | CA15-3 | Relatively specific to breast cancer, with low positive rate in early stage |
| | CEA | No organ specificity, with false positivity |
| | NCC-ST-439 | May not be produced in highly malignant cases |
| Colorectal cancer | CEA | No organ specificity, with false positivity |
| | CA19-9 | Only for late stage cancer |
| | p53 antibody | No organ specificity, with high positive rate for cancer |
| Lung cancer | CEA | No organ specificity, with false positivity |
| Lung cancer (small-cell carcinoma) | proGRP | Relatively high specificity, with low sensitivity |
| | NSE | Relatively high specificity, with low sensitivity |
| Lung cancer (squamous-cell carcinoma) | CYFRA | Relatively high specificity, with low sensitivity |
| | SCC | Relatively high specificity, with low sensitivity |
| Lung cancer (adenocarcinoma) | SLX | Relatively low false positivity |

As an aside, AZU1 is an inactive serine protease, also known as heparin-binding protein (HBP) or 37-kDa cationic antimicrobial protein (CAP37). AZU1 has a chemoattracting effect on monocytes and an antimicrobial activity against Gram-negative bacteria as its functions. AZU1 is present in azurophilic granules of neutrophils and is released from neutrophils that have migrated to the site of infection, thereby inducing vascular leakage and edema formation, promoting inflammation, and contributing to host defense (Non-patent Documents 1 to 5).

Regarding the association between AZU1 and diseases, a method for diagnosing infectious diseases and sepsis by measuring the AZU1 level in body fluid has already been disclosed (Patent Documents 1 to 3). Further, in recent years, a method for diagnosing renal cell cancer by isolating extracellular vesicles in body fluid and detecting AZU1 has been reported (Patent Document 4 and Non-patent Document 6).

Regarding the association between AZU1 and cancer, it has been reported that the expression level of messenger RNA of AZU1 is increased in breast cancer, prostate cancer, and colorectal cancer (Non-patent Document 6). However, so far, there have been no reports on the dynamics of AZU1 in body fluid that can be collected less invasively than biopsy samples in cancers, including these cancers excluding renal cell cancer. It has been unknown whether AZU1 in body fluid can be applied to detect cancers, excluding renal cell cancer.

### PRIOR ART DOCUMENTS

### Patent Documents

Patent Document 1: Japanese Patent No. 5166522
Patent Document 2: Japanese Patent No. 5488885
Patent Document 3: Japanese Patent No. 5818916
Patent Document 4: WO2018/079689

### Non-patent Documents

Non-patent Document 1: J Leukoc Biol. 2009 Mar;85(3):344-51
Non-patent Document 2: Trends Immunol. 2009 Nov;30(11):538-46
Non-patent Document 3: Nat Med. 2001 Oct;7(10):1123-7.
Non-patent Document 4: Trends Immunol. 2009 Nov;30(11):547-56
Non-patent Document 5: Thromb Haemost. 2009 Aug; 102 (2) :198-205
Non-patent Document 6: Int J Cancer. 2018 Feb 1;142(3):607-617

### SUMMARY OF THE INVENTION

### PROBLEMS TO BE SOLVED BY THE INVENTION

An object of the present invention is to provide a method for detecting cancer in a simple and highly accurate manner and a reagent that can be used in the method.

### MEANS FOR SOLVING THE PROBLEMS

The present inventors have made intensive studies to solve the above-described problems. As a result, the present inventors have found out that AZU1 in body fluid is significantly higher in cancer patients than in healthy individuals. Based on these findings, the present inventors discovered that AZU1 can be a cancer detection marker, thereby completing the present invention.

That is, the present invention encompasses the following aspects.
[1] A method for detecting cancer (excluding renal cell cancer), which comprises measuring the level of Azurocidin (AZU1) in a sample, wherein it is determined that cancer is detected when a measured value exceeds a preset reference value.
[2] The method according to [1], wherein the cancer is selected from the group consisting of stomach cancer, breast cancer, colorectal cancer, and lung cancer.
[3] The method according to [1] or [2], wherein the level of AZU1 is measured using an antibody that specifically recognizes AZU1.
[4] The method according to any one of [1] to [3], which comprises further detecting a second marker present on cell-secreted fine particles that are cell-secreted fine particles on which AZU1 is present in the sample, wherein the second marker is at least one of second markers listed in Table 2.
[5] The method according to [4], wherein the second marker contains at least one of CD81, CD63, CD9, and phosphatidylserine.
[6] The method according to [4] or [5], wherein the second marker is detected using an antibody or receptor that specifically recognizes the second marker.
[7] A reagent for use in detecting cancer (excluding renal cell cancer), which contains an antibody that specifically recognizes AZU1.
[8] The reagent according to [7], wherein the cancer is selected from the group consisting of stomach cancer, breast cancer, colorectal cancer, and lung cancer.
[9] The reagent according to [7] or [8], which further contains an antibody or receptor that specifically recognizes any of second markers listed in Table 2.
[10] The reagent according to [9], wherein the second marker contains at least one of CD81, CD63, CD9, and phosphatidylserine.

### EFFECT OF THE INVENTION

The present invention provides a method for detecting cancer in a simple and highly accurate manner and a reagent that can be used in the method.

### BRIEF DESCRIPTION OF THE DRAWINGS

FIG. 1 is a diagram showing the results of screening of hybridoma cell culture supernatant by CELISA using CHO-K1 cells constitutively expressing GPI-anchor type AZU1 in Example 8.
FIG. 2 is a diagram showing the results of screening of hybridoma cell culture supernatant by ELISA using secretory AZU1 in Example 9.
FIG. 3 is a diagram showing the results of performance evaluation of anti-AZUl monoclonal antibody as a solid-phase antibody by ELISA using cell-secreted fine particles in Example 13.
FIG. 4 is a diagram showing the results of performance evaluation of anti-AZUl monoclonal antibody as a biotin-labeled antibody by ELISA using cell-secreted fine particles in Example 13.
FIG. 5 is a diagram showing that cell-secreted fine particles containing AZU1 can be detected by using any of an anti-CD81 antibody, an anti-CD9 antibody, and an anti-CD63 antibody as the solid-phase antibody of ELISA in Example 14.
FIG. 6 is a diagram showing box plots of ELISA absorbance using an anti-CD81 antibody as a solid-phase antibody and an anti-AZUl antibody as a biotin-labeled antibody in a healthy individual group and in a stomach cancer patient group in Example 15-1.
FIG. 7 is a diagram showing box plots of ELISA absorbance using an anti-CD9 antibody as a solid-phase antibody and an anti-AZU1 antibody as a biotin-labeled antibody in a healthy individual group and in a stomach cancer patient group in Example 15-2.
FIG. 8 is a diagram showing box plots of ELISA absorbance using an anti-CD63 antibody as a solid-phase antibody and an anti-AZUl antibody as a biotin-labeled antibody in a healthy individual group and in a stomach cancer patient group in Example 15-3.
FIG. 9 is a diagram showing box plots of ELISA absorbance using an anti-AZUl antibody as a solid-phase antibody and an anti-CD81 antibody as a biotin-labeled antibody in a healthy individual group and in a stomach cancer patient group in Example 15-4.
FIG. 10 is a diagram showing box plots of ELISA absorbance using an anti-AZUl antibody as a solid-phase antibody and an anti-CD9 antibody as a biotin-labeled antibody in a healthy individual group and in a stomach cancer patient group in Example 15-5.
FIG. 11 is a diagram showing box plots of ELISA absorbance using an anti-AZUl antibody as a solid-phase antibody and an anti-CD63 antibody as a biotin-labeled antibody in a healthy individual group and in a stomach cancer patient group in Example 15-6.
FIG. 12 is a diagram showing box plots of ELISA absorbance using Tim4-hFc as a solid-phase receptor and an anti-AZUl antibody as a biotin-labeled antibody in a healthy individual group and in a stomach cancer patient group in Example 16.
FIG. 13 is a diagram showing box plots of measured values of CEA in a healthy individual group and in a stomach cancer patient group in Comparative Example 1.
FIG. 14 is a diagram showing the results of receiver operating characteristic (ROC) curve analysis of ELISA absorbance using an anti-CD81 antibody as a solid-phase antibody and an anti-AZUl antibody as a biotin-labeled antibody for discrimination between a healthy individual group and a stomach cancer patient group in Example 17.
FIG. 15 is a diagram showing the results of ROC curve analysis of ELISA absorbance using an anti-CD9 antibody as a solid-phase antibody and an anti-AZUl antibody as a biotin-labeled antibody for discrimination between a healthy individual group and a stomach cancer patient group in Example 17.
FIG. 16 is a diagram showing the results of ROC curve analysis of ELISA absorbance using an anti-CD63 antibody as a solid-phase antibody and an anti-AZUl antibody as a biotin-labeled antibody for discrimination between a healthy individual group and a stomach cancer patient group in Example 17.
FIG. 17 is a diagram showing the results of ROC curve analysis of ELISA absorbance using an anti-AZUl antibody as a solid-phase antibody and an anti-CD81 antibody as a biotin-labeled antibody for discrimination between a healthy individual group and a stomach cancer patient group in Example 17.
FIG. 18 is a diagram showing the results of ROC curve analysis of ELISA absorbance using an anti-AZUl antibody as a solid-phase antibody and an anti-CD9 antibody as a biotin-labeled antibody for discrimination between a healthy individual group and a stomach cancer patient group in Example 17.
FIG. 19 is a diagram showing the results of ROC curve analysis of ELISA absorbance using an anti-AZUl antibody as a solid-phase antibody and an anti-CD63 antibody as a biotin-labeled antibody for discrimination between a healthy individual group and a stomach cancer patient group in Example 17.
FIG. 20 is a diagram showing the results of ROC curve analysis of ELISA absorbance using Tim4-hFc as a solid-phase receptor and an anti-AZUl antibody as a biotin-labeled antibody for discrimination between a healthy individual group and a stomach cancer patient group in Example 17.
FIG. 21 is a diagram showing the results of ROC curve analysis of measured values of CEA for discrimination between a healthy individual group and a stomach cancer patient group in Example 17.
FIG. 22 is a diagram showing box plots of ELISA absorbance using an anti-CD81 antibody as a solid-phase antibody and an anti-AZUl antibody as a biotin-labeled antibody in a healthy individual group and in a breast cancer patient group in Example 18-1.
FIG. 23 is a diagram showing box plots of ELISA absorbance using an anti-CD9 antibody as a solid-phase antibody and an anti-AZU1 antibody as a biotin-labeled antibody in a healthy individual group and in a breast cancer patient group in Example 18-2.
FIG. 24 is a diagram showing box plots of ELISA absorbance using an anti-CD63 antibody as a solid-phase antibody and an anti-AZUl antibody as a biotin-labeled antibody in a healthy individual group and in a breast cancer patient group in Example 18-3.
FIG. 25 is a diagram showing box plots of ELISA absorbance using an anti-CD81 antibody as a solid-phase antibody and an anti-AZUl antibody as a biotin-labeled antibody in a healthy individual group and in a colorectal cancer patient group in Example 19-1.
FIG. 26 is a diagram showing box plots of ELISA absorbance using an anti-CD9 antibody as a solid-phase antibody and an anti-AZU1 antibody as a biotin-labeled antibody in a healthy individual group and in a colorectal cancer patient group in Example 19-2.
FIG. 27 is a diagram showing box plots of ELISA absorbance using an anti-CD63 antibody as a solid-phase antibody and an anti-AZUl antibody as a biotin-labeled antibody in a healthy individual group and in a colorectal cancer patient group in Example 19-3.
FIG. 28 is a diagram showing box plots of ELISA absorbance using an anti-CD81 antibody as a solid-phase antibody and an anti-AZUl antibody as a biotin-labeled antibody in a healthy individual group and in a lung cancer patient group in Example 20-1.
FIG. 29 is a diagram showing box plots of ELISA absorbance using an anti-CD9 antibody as a solid-phase antibody and an anti-AZU1 antibody as a biotin-labeled antibody in a healthy individual group and in a lung cancer patient group in Example 20-2.
FIG. 30 is a diagram showing box plots of ELISA absorbance using an anti-CD63 antibody as a solid-phase antibody and an anti-AZUl antibody as a biotin-labeled antibody in a healthy individual group and in a lung cancer patient group in Example 20-3.
FIG. 31 is a diagram showing box plots of free AZU1 concentration in serum samples in healthy individual, lung cancer, colorectal cancer, breast cancer, and stomach cancer groups in Example 21.

### MODE FOR CARRYING OUT THE INVENTION

### [1] Method for Detecting Cancer According to Present Invention

A first aspect of the present invention is a method for detecting cancer (excluding renal cell cancer), which comprises measuring the AZU1 level in a sample. This is a method based on the fact that AZU1 is characteristically present in a biological sample, such as blood, of an individual with cancer, unlike in a sample of a healthy individual. Measurement of the AZU1 level in a sample is usually performed *in vitro.*

According to the method of the present invention, as shown in the Examples described later, cancer can be detected with higher sensitivity and specificity than when a conventionally known tumor marker such as CEA is measured.

The method of the present invention includes detecting cancer as a final step, and does not include the action to make a final decision on cancer diagnosis. A physician diagnoses cancer and formulates a treatment policy by referring to the detection results and the like by the method of the present invention.

Usually, the target (subject animal) for detecting cancer is a human.

Examples of a sample to be measured in the present invention include blood, urine, saliva, tears, ascites, peritoneal lavage fluid, cerebrospinal fluid, and cell or tissue extract. Blood, urine, saliva, and tears are preferable in consideration of the ease of sample collection. Blood is more preferable given its versatility for other test items. Blood may be used as whole blood or separated into blood components such as serum, plasma, and blood cells, but serum or plasma is preferably used. The dilution ratio of the sample is not particularly limited, but for example, it may be appropriately selected in the range of undiluted to 100-fold dilution according to the type and state of the sample to be used.

The sample usually contains fine particles (cell-secreted fine particles) secreted from cells, which will be described later.

The disease targeted by the present invention is cancer (excluding renal cell cancer). Stomach cancer, breast cancer, colorectal cancer, and/or lung cancer are preferable, and stomach cancer is more preferable.

AZU1 to be measured in the present invention is a peptide containing the sequence from isoleucine at the 27th residue to proline at the 248th residue of the amino acid sequence of the human AZU1 protein disclosed in Accession No. P20160 of UniPlotKB or a peptide containing an amino acid sequence having 80% or more identity with the above-described sequence. The identity is preferably 90% or more, more preferably 95% or more. The peptide may also be a peptide consisting of amino acids in which one or more amino acids have been deleted, substituted, inserted, or added in the above-described sequence. The word "more" refers to preferably from 2 to 20, more preferably from 2 to 10, and even more preferably from 2 to 5. In addition, other peptide fragments may be provided at both termini of the sequence.

The AZU1 to be measured in the present invention may be measured as AZU1 present as a soluble protein, AZU1 present on fine particles secreted from cells, or both thereof. When measuring AZU1 present on fine particles, AZU1 coexisting with a second marker on the fine particles may be measured.

Examples of fine particles secreted from cells include exosomes. Exosomes are membrane vesicles composed of a lipid bilayer membrane, usually having a diameter of from 50 to 200 nm. Exosomes are known to contain a large amount of membrane proteins such as tetraspanins and integrins, proteins related to multivesicular body formation, and heat-shock proteins. Further, it is known that the lipid bilayer membrane constituting an exosome has phosphatidylserine on the membrane surface. Table 2 shows typical molecules that are abundant in exosomes.

The second marker in the present invention is not particularly limited as long as it is a molecule present on cell-secreted fine particles but preferably refers to at least one included in the group consisting of the above-described proteins and phosphatidylserine listed in Table 2, including more preferably at least one of CD81, CD63, CD9, and phosphatidylserine. It is assumed that the proteins listed in Table 2 also include peptides containing an amino acid sequence having high homology (80% or more, preferably 90% or more, more preferably 95% or more) thereto.

As the second marker may include two or more types thereof, the word "second" may be understood to mean "other than AZU1."

The second marker to be detected in the present invention is not particularly limited as long as it is a molecule present on cell-secreted fine particles but is preferably the proteins and phosphatidylserine listed in Table 2, and the second marker is present on cell-secreted fine particles that are cell-secreted fine particles on which AZU1 is present.

### [Table 2-1]

**(Table 2-1)**

| **Protein (78 types) (Page 1/2)** | | | |
|---|---|---|---|
| | (Gene Name) | (Gene Symbol) | (UniPlotKB) |
| 1 | actin gamma 1 | ACTG1 | P63261 |
| 2 | actin, beta | ACTB | P60709 |
| 3 | albumin | ALB | P02768 |
| 4 | aldolase A, fructose-bisphosphate | ALDOA | P04075 |
| 5 | annexin A2 | ANXA2 | P07355 |
| 6 | annexin A5 | ANXA5 | P08758 |
| 7 | caveolin 1, caveolae protein, 22kDa | CAV1 | Q03135 |
| 8 | caveolin 2 | CAV2 | P51636 |
| 9 | CD151 antigen | CD151 | P48509 |
| 10 | CD24 molecule | CD24 | P25063 |
| 11 | CD40 molecule, TNF receptor superfamily member 5 | CD40 | P25942 |
| 12 | CD44 molecule (Indian blood group) | CD44 | P16070 |
| 13 | CD58 molecule | CD58 | P19256 |
| 14 | CD63 molecule | CD63 | P08962 |
| 15 | CD81 molecule | CD81 | P60033 |
| 16 | CD82 molecule | CD82 | P27701 |
| 17 | CD9 molecule | CD9 | P21926 |
| 18 | chemokine (C-X-C motif) receptor 4 | CXCR4 | P61073 |
| 19 | cofilin 1 (non-muscle) | CFL1 | P23528 |
| 20 | enolase 1, (alpha) | ENOl | P06733 |
| 21 | epithelial cell adhesion molecule | EPCAM | P16422 |
| 22 | eukaryotic translation elongation factor 1 alpha 1 | EEF1A1 | P68104 |
| 23 | eukaryotic translation elongation factor 2 | EEF2 | P13639 |
| 24 | flotillin 1 | FLOT1 | 075955 |
| 25 | flotillin 2 | FLOT2 | Q14254 |
| 26 | glyceraldehyde-3-phosphate dehydrogenase | GAPDH | P04406 |
| 27 | heat shock 70kDa protein 1A | HSPA1A | P08107 |
| 28 | heat shock 70kDa protein 5 (glucose-regulated protein, 78kDa) | HSPA5 | P11021 |
| 29 | heat shock 70kDa protein 8 | HSPA8 | P11142 |
| 30 | heat shock protein 90kDa alpha (cytosolic), class A member 1 | HSP90AA1 | P07900 |
| 31 | heat shock protein 90kDa alpha (cytosolic), class B member 1 | HSP90AB1 | P08238 |
| 32 | integrin, alpha 2b (platelet glycoprotein IIb of IIb/IIIa complex, antigen CD41) | ITGA2B | P08514 |
| 33 | intercellular adhesion molecule 1 | ICAM1 | P05362 |
| 34 | lactate dehydrogenase A | LDHA | P00338 |
| 35 | leukocyte antigen CD37 | CD37 | P11049 |
| 36 | leukocyte surface antigen CD53 | CD53 | P19397 |
| 37 | milk fat globule-EGF factor 8 protein | MFGE8 | Q08431 |
| 38 | moesin | MSN | P26038 |
| 39 | peripherin-2 | PRPH2 | P23942 |
| 40 | phosphoglycerate kinase 1 | PGK1 | P00558 |

### [Table 2-2]

**(Table 2-2)**

| **Protein (78 types) (Page 2/2)** | | | |
|---|---|---|---|
| | (Gene Name) | (Gene Symbol) | (UniPlotKB) |
| 41 | programmed cell death 6 interacting protein | PDCD6IP | Q8WUM4 |
| 42 | putative tetraspanin-19 | TSPAN19 | P0C672 |
| 43 | pyruvate kinase, muscle | PKM | P14618 |
| 44 | RAB5A, member RAS oncogene family | RAB5A | P20339 |
| 45 | RAB7A, member RAS oncogene family | RAB7A | P51149 |
| 46 | rod outer segment membrane protein 1 | ROM1 | Q03395 |
| 47 | selectin L | SELL | P14151 |
| 48 | selectin P (granule membrane protein 140kDa, antigen CD62) | SELP | P16109 |
| 49 | syndecan binding protein (syntenin) | SDCBP | 000560 |
| 50 | tetraspanin-1 | TSPAN1 | 060635 |
| 51 | tetraspanin-10 | TSPAN10 | Q9H1Z9 |
| 52 | tetraspanin-11 | TSPAN11 | A1L157 |
| 53 | tetraspanin-12 | TSPAN12 | 095859 |
| 54 | tetraspanin-13 | TSPAN13 | 095857 |
| 55 | tetraspanin-14 | TSPAN14 | Q8NG11 |
| 56 | tetraspanin-15 | TSPAN15 | 095858 |
| 57 | tetraspanin-16 | TSPAN16 | Q9UKR8 |
| 58 | tetraspanin-17 | TSPAN17 | Q96FV3 |
| 59 | tetraspanin-18 | TSPAN18 | Q96SJ8 |
| 60 | tetraspanin-2 | TSPAN2 | 060636 |
| 61 | tetraspanin-3 | TSPAN3 | 060637 |
| 62 | tetraspanin-31 | TSPAN31 | Q12999 |
| 63 | tetraspanin-32 | TSPAN32 | Q96QS1 |
| 64 | tetraspanin-33 | TSPAN33 | Q86UF1 |
| 65 | tetraspanin-4 | TSPAN4 | 014817 |
| 66 | tetraspanin-5 | TSPAN5 | P62079 |
| 67 | tetraspanin-6 | TSPAN6 | 043657 |
| 68 | tetraspanin-7 | TSPAN7 | P41732 |
| 69 | tetraspanin-8 | TSPAN8 | P19075 |
| 70 | tetraspanin-9 | TSPAN9 | 075954 |
| 71 | tissue specific transplantation antigen P35B | TSTA3 | Q13630 |
| 72 | transmembrane 9 superfamily protein member 4 | TM9SF4 | Q92544 |
| 73 | tumor susceptibility 101 | TSG101 | Q99816 |
| 74 | tyrosine 3-monooxygenase/tryptophan 5-monooxygenase activation protein, epsilon | YWHAE | P62258 |
| 75 | tyrosine 3-monooxygenase/tryptophan 5-monooxygenase activation protein, zeta | YWHAZ | P63104 |
| 76 | uroplakin-1a | UPK1A | 000322 |
| 77 | uroplakin-1b | UPK1B | 075841 |
| 78 | vimentin | VIM | P08670 |

| **Lipid (1 type)** | | | |
|---|---|---|---|
| | Phosphatidylserine | | |

In the direction method of the present invention, the method for measuring the AZU1 level and the method for measuring (detecting) at least one of second markers and AZU1 coexisting on cell-secretory fine particles are not particularly limited unless the measurement of the AZU1 level is not interrupted. For example, an immunoassay method using an antibody that specifically recognizes AZU1 and a method using mass spectrometry can be mentioned.

Specific examples of the immunoassay method using an antibody that specifically recognizes AZU1 include the following.

[a] A competition method in which an antibody that specifically recognizes AZU1 and labeled AZU1 are used and which utilizes the competitive binding of the labeled AZU1 and AZU1 contained in the sample to the antibody.
[b] A method using surface plasmon resonance, in which the sample is brought into contact with a chip on which an antibody that specifically recognizes AZU1 is immobilized, and a signal dependent on the binding of the antibody to AZU1 is detected.
[c] A fluorescence polarization immunoassay in which a fluorescence-labeled antibody that specifically recognizes AZU1 is used, and which utilizes the phenomenon that the binding of the antibody to AZU1 causes an increase in the degree of fluorescence polarization.
[d] A sandwich method in which two antibodies that specifically recognize AZU1 (one of which is a labeled antibody) are used so as to allow the formation of a complex of the three, namely, a complex of the two antibodies and AZU1. At this time, the two antibodies are preferably two types of antibodies having different epitopes.
[e] A method in which AZU1 in the sample is concentrated by an antibody that specifically recognizes AZU1 as a pretreatment, and then the binding product of AZU1 and the antibody is detected using a mass spectrometer.
[f] A flow cytometry method in which a fluorescence-labeled antibody that specifically recognizes AZU1 is used, the antibody is bounded to AZU1 in measuring objects, the measuring objects are aligned in the fluid stream, and then the number of the complexes of the antibody and the measuring objects is counted based on the scattered light and fluorescence from the individual particles obtained when irradiated with excitation light.

Although the methods [d] and [e] among the above are simple and highly versatile, the method [d] is more preferred for processing a large number of samples since the technologies related to the reagents and the devices used in this method have been sufficiently established.

The antibody that specifically recognizes AZU1 is not particularly limited but can be obtained by immunizing an animal using, as an immunogen, the AZU1 protein itself, an oligopeptide consisting of a partial region of AZU1, a polynucleotide encoding the full length or partial region of AZU1, or the like.

Note that, in cases where the AZU1 protein itself or an oligopeptide composed of a partial region of the AZU1 protein is used as the immunogen, the structure of the protein or the oligopeptide may change during the preparation process thereof. Therefore, the resulting antibody may not have a high specificity or binding capacity to the desired antigen, in some cases, possibly resulting in a failure to quantify the level of AZU1 contained in the sample accurately. On the other hand, in cases where an expression vector containing a polynucleotide encoding the full length or partial region of AZU1 is used as the immunogen, AZU1 is expressed as it is, without undergoing a structural change in the body of the immunized animal.

Therefore, an antibody having high specificity and binding capacity (namely, a high affinity) to the desired antigen can be obtained, which is preferred.

The animal to be used for the immunization is not particularly limited as long as the animal has the ability to produce antibodies. The animal may be a mammal normally used for immunization, such as a mouse, rat, or rabbit, or may be a bird such as a chicken.

The antibody that specifically recognizes AZU1 may be either a monoclonal antibody or a polyclonal antibody. The antibody is preferably a monoclonal antibody.

The establishment of a hybridoma cell that produces an antibody that specifically recognizes AZU1 can be carried out by a method selected as appropriate from methods whose techniques have been established. For example, a hybridoma cell that produces a monoclonal antibody that specifically recognizes AZU1 can be established by collecting B cells from an animal immunized by the above method, fusing the B cells with myeloma cells electrically or in the presence of polyethylene glycol, selecting a hybridoma cell that produces the desired antibody using HAT medium, and cloning the selected hybridoma cell by the limiting dilution method.

The antibody that specifically recognizes AZU1 used in the present invention may be selected based on the affinity for glycosylphosphatidylinositol (GPI)-anchor type AZU1 derived from a host expression system.

Note that the host is not particularly limited and can be selected as appropriate from cells of microorganisms such as E. coli and yeast, insect cells, and animal cells that are usually used for protein expression by those skilled in the art. The host is preferably a mammalian cell since it enables the expression of a protein having a structure similar to that of natural AZU1 by post-translational modification such as disulfide bonding or glycosylation. Examples of mammalian cells include the human embryonic kidney-derived 293T cell line, monkey kidney-derived COS-7 cell line, Chinese hamster ovary-derived CHO-K1 cells, and cancer cells isolated from humans, which are conventionally used.

The purification of the antibody to be used in the method for detecting cancer according to the present invention can be carried out by a method selected as appropriate from methods whose techniques have been established. For example, after culturing hybridoma cells which are established by the above method and which produce an antibody, the culture supernatant may be collected, and the antibody may be concentrated, if necessary, by ammonium sulfate precipitation. Thereafter, ion-exchange chromatography, hydrophobic interaction chromatography, or affinity chromatography using a carrier to which Protein A, Protein G, Protein L, or the like is immobilized can be carried out to achieve the purification of the antibody.

In the present invention, in a case where both the measurement of the level of AZU1 present on cell-secreted fine particles and the detection of a second marker present on the same are performed, it is preferable to further use an antibody or receptor that specifically recognizes the second marker (hereinafter, also referred to as "antibody or the like") as well as the antibody that recognizes AZU1.

In the present invention, the second marker preferably contains at least one of CD81, CD63, CD9, and phosphatidylserine. The antibody or receptor that specifically recognizes them is preferably an anti-CD81 antibody, an anti-CD63 antibody, an anti-CD9 antibody, or a phosphatidylserine receptor.

The anti-CD81 antibody, anti-CD63 antibody, and anti-CD9 antibody used in the present invention can be obtained by the same method as the above-described antibody that recognizes AZU1.

The phosphatidylserine receptor used in the present invention is not particularly limited, and examples thereof include Annexin V, MFG-E8, Tim1, Tim3, and Tim4, and Tim4, having high specificity and binding capacity to phosphatidylserine (WO2016/088689) is preferable. Tim4 may have at least an amino acid sequence of a binding domain (IgV domain) for phosphatidylserine. For example, a Tim4 protein itself, a peptide consisting of a partial region containing the IgV domain of Tim4, or a fusion protein in which another peptide fragment is bound to a partial region containing the IgV domain of Tim4 can be used.

Note that a labeled antibody or the like used when performing the binding quantification method by the sandwich method described above can be labeled with enzymes such as peroxidase and alkaline phosphatase, substances detectable by detection devices, such as fluorescent substances, chemiluminescent substances, radioisotopes, and functional fine particles, and substances to which another molecule specifically binds, such as biotin, to which avidin specifically binds. The labeling may also be performed using a well-established technique.

The method for detecting and quantitatively measuring AZU1 by using mass spectrometry according to the present invention will be specifically described below.

In the case of using blood as a sample, it is preferred that proteins such as albumin, immunoglobulin, and transferrin, which are contained in large amounts in the blood, be removed as a pretreatment step, using Agilent Human 14 or the like, followed by further fractionation by ion exchange, gel filtration, reverse-phase chromatography, and/or the like.

The measurement can be carried out by tandem mass spectrometry (MS/MS), liquid chromatography-tandem mass spectrometry (LC/MS/MS), matrix-assisted laser desorption ionization time-of-flight mass spectrometry (MALDI-TOF/MS), surface-enhanced laser desorption ionization mass spectrometry (SELDI-MS), or the like.

In the detection method according to the present invention, it is preferred to determine that cancer is detected when the AZU1 level obtained by the measurement is higher than a reference value (cutoff value) calculated from a control.

The AZU1 level to be used for the determination may be either a measured value or a converted concentration value. Note that the converted concentration value refers to a value converted from a measured value based on a calibration curve prepared using AZU1 as a standard sample. The concentration of the standard sample may be determined as a value converted from a measured value based on a calibration curve of a standard peptide, prepared using mass spectrometry.

The cutoff value may be set as appropriate to a measured value which provides optimum sensitivity and specificity based on the receiver operating characteristic (ROC) curve constructed from measured values of samples from healthy individuals and that from cancer patients.

The method for detecting cancer of the present invention can be applied to a method for treating cancer. That is, according to the present invention, a method for treating cancer (excluding renal cell cancer) in a patient, which comprises:
(i) a step of identifying a patient as having a measured value of the AZU1 level that exceeds a preset reference value; and
(ii) a step of treating the identified patient, is provided.

Upon the identification of step (i), the AZU1 level may be measured by using an antibody that specifically recognizes AZU1 or by using mass spectrometry.

The treatment of step (ii) includes, but is not limited to, surgical resection, drug therapy, radiation therapy, and the like.

### [2] Reagent for Detecting Cancer according to Present Invention

A second aspect of the present invention is a reagent for detecting cancer (excluding renal cell cancer), which contains an antibody that specifically recognizes AZU1.

It is preferable that the reagent of the present invention further contains an antibody or receptor that specifically recognizes a second marker listed in Table 2. The antibody or receptor (antibody or the like) that specifically recognizes the second marker is not particularly limited. For example, the antibody or the like is preferably an antibody or receptor that specifically recognizes at least one of CD81, CD63, CD9, and phosphatidylserine, and more preferably an anti-CD81 antibody, an anti-CD63 antibody, an anti-CD9 antibody, or a phosphatidylserine receptor. The phosphatidylserine receptor is not particularly limited, and examples thereof include Annexin V, MFG-E8, Tim1, Tim3, and Tim4, and Tim4 having high specificity and binding capacity to phosphatidylserine is preferable. Tim4 may have at least an amino acid sequence of a binding domain (IgV domain) for phosphatidylserine. For example, a Tim4 protein itself, a peptide consisting of a partial region containing the IgV domain of Tim4, or a fusion protein in which another peptide fragment is bound to a partial region containing the IgV domain of Tim4 can be used.

The reagent and the measurement method of the present invention will be specifically described below with respect to the three modes of the sandwich method described above. However, the reagent and the measurement method of the present invention are not limited to these three modes.

### [Mode 1] Two-Step Sandwich Method

The reagent used in this mode contains two types of antibodies or the like (hereinafter, referred to as "Antibody or the like 1" and "Antibody or the like 2"). It is preferable that the antibody or the like 1 and the antibody or the like 2 have different binding sites for the substance to be measured. Examples of the combination of the antibody or the like 1 and the antibody or the like 2 include the following three combinations [a] to [c] .
[a] Antibody or the like 1: Antibody that specifically recognizes AZU1; Antibody or the like 2: Antibody that specifically recognizes AZU1 and is the same as or different from Antibody or the like 1
[b] Antibody or the like 1: Antibody that specifically recognizes AZU1; Antibody or the like 2: Antibody or receptor that specifically recognizes the second marker
[c] Antibody or the like 1: Antibody or receptor that specifically recognizes the second marker; Antibody or the like 2: Antibody that specifically recognizes AZU1

The reagent of this mode can be prepared by the method described in the following [1] to [3].
[1] Antibody or the like 1 is first bound to a carrier capable of B/F (Bound/Free) separation, such as an immuno-plate or magnetic particles. Antibody or the like 1 may be physically bound to the carrier utilizing hydrophobic bonding or may be chemically bound thereto using, for example, a linker reagent capable of cross-linking two substances to each other.
[2] After binding the Antibody or the like 1 to the carrier, the surface of the carrier is subjected to a blocking treatment using bovine serum albumin, skim milk, a commercially available immunoassay blocking reagent, or the like, for preventing non-specific binding, to provide a primary reagent.
[3] The other antibody, Antibody or the like 2, is then labeled, and a solution containing the resulting labeled antibody is prepared as a secondary reagent. Preferred examples of the substance to be used for labeling the Antibody or the like 2 include enzymes such as peroxidase and alkaline phosphatase; substances detectable by detection devices, such as fluorescent substances, chemiluminescent substances, radioisotopes, and functional fine particles; and substances to which another molecule specifically binds, such as biotin, to which avidin specifically binds. Preferred examples of the solution to be used for the secondary reagent include buffers that allow an antigen-antibody reaction to proceed favorably, such as phosphate buffer and Tris-HCl buffer.

The thus prepared reagent of this mode may be freeze-dried, if necessary.

Next, for the detection and measurement of AZU1 using the reagent prepared by this mode, the method described in the following [4] to [6] can be carried out.

[4] The primary reagent prepared in [2] described above is brought into contact with a sample for a predetermined period of time at a constant temperature. The reaction can be carried out under the conditions of a temperature within the range of from 4°C to 40°C for from 5 minutes to 180 minutes.

[5] Unreacted substances are removed by B/F separation, and then the secondary reagent prepared in [3] is brought into contact with the reaction product for a predetermined period of time at a constant temperature to allow the formation of a sandwich complex. The reaction can be carried out under the conditions of a temperature within the range of from 4°C to 40°C for from 5 minutes to 180 minutes.

[6] Unreacted substances are removed by B/F separation, and the labeling substance of the labeled antibody or the like is quantified. Based on a calibration curve prepared using known concentrations of AZU1 as standards, the concentration of AZU1 in the sample is quantified.

### [Mode 2] One-Step Sandwich Method

The reagent used in this mode contains Antibody or the like 1 and Antibody or the like 2 in the same manner as in Mode 1 described above.

The binding of Antibody or the like 1 to the carrier and blocking treatment can be carried out in the same manner as in [1] and [2] of Mode 1, and a buffer containing labeled Antibody or the like 2 can be further added to the carrier to which the antibody or the like is immobilized to prepare a reagent of this mode.

The thus prepared reagent of this mode may be freeze-dried, if necessary.

Next, for the detection and measurement of AZU1 using the reagent prepared by this mode, the method described in the following [7] and [8] can be carried out.

[7] The reagent prepared by the method described above is brought into contact with a sample for a predetermined period of time at a constant temperature so as to form a sandwich complex. The reaction can be carried out under the conditions of a temperature within the range of from 4°C to 40°C for from 5 minutes to 180 minutes.

[8] Unreacted substances are removed by B/F separation, and the labeling substance of the labeled antibody or the like is quantified. Based on a calibration curve prepared using known concentrations of AZU1 as standards, the concentration of AZU1 in the sample is quantified.

### [Mode 3] Homogeneous Sandwich Method

The reagent used in this mode contains Antibody or the like 1 and Antibody or the like 2 in the same manner as in Modes 1 and 2 described above, and further contains a streptavidin-coated labeling substance that is excited by excitation light.

As the streptavidin-coated labeling substance, for example, AlphaScreen streptavidin donor beads (manufactured by PerkinElmer) can be preferably used.

The reagent of this mode can be prepared by the method described in the following [9] and [10].

[9] First, Antibody or the like 1 is labeled with biotin. The biotin labeling may be carried out by a conventionally known method, and examples thereof include a method using a Biotin labeling Kit-NH2 labeling kit (manufactured by Dojindo Laboratories).

[10] The other antibody, Antibody or the like 2, is labeled with a substance that emits a signal by receiving singlet oxygen. This singlet oxygen is generated when the streptavidin-coated labeling substance is excited. The signal is preferably a fluorescent signal. As the signal generating substance, for example, AlphaLISA acceptor beads (manufactured by PerkinElmer) can be preferably used. The method for binding Antibody or the like 2 to the signal generating substance is not particularly limited, and examples thereof include reductive amination cross-linking to the aldehyde group on the surface of the signal generating substance using sodium cyanoborohydride.

Next, for the detection and measurement of AZU1 using the reagent prepared by this mode, the method described in the following [11] to [13] can be carried out.

[11] The biotin-labeled antibody or the like 1 prepared in [9] described above and the signal-generating substance-labeled antibody or the like 2 prepared in [10] are brought into contact with a sample under shading conditions for a predetermined period of time at a constant temperature so as to form a sandwich complex. The reaction can be carried out under the conditions of a temperature within the range of from 4°C to 40°C for from 5 minutes to 180 minutes.

[12] Subsequently, a streptavidin-coated labeling substance is added to be in contact with the complex under shading conditions for a certain period of time at a constant temperature so as to bind the biotin-labeled antibody and the streptavidin-coated labeling substance. The reaction can be carried out under the conditions of a temperature within the range of from 4°C to 40°C for from 5 minutes to 180 minutes.

[13] The signal emitted from the signal-generating substance-labeled Antibody or the like 21 when irradiated with excitation light using an analyzer is quantified. Based on a calibration curve prepared using known concentrations of AZU1 as standards, the concentration of AZU1 in the sample is quantified. As the analyzer, for example, EnSpire (manufactured by PerkinElmer) can be preferably used.

The amount of each reagent component contained in the reagent of the present invention can be set as appropriate depending on the conditions such as the amount of the sample, the type of the sample, the type of the reagent, and the measurement method. For example, in cases where the AZU1 level is measured by a sandwich method using 20 µL of serum or plasma as a sample, the amount of Antibody or the like 1 to be bound to the carrier may be from 100 ng to 1,000 µg, and the amount of the labeled Antibody or the like 2 may be from 2 ng to 20 µg in a reaction system in which 20 µL of the sample is reacted with the antibodies or the like.

The reagent according to the present invention is applicable to either manual measurement or measurement using an automatic immunodiagnostic apparatus. In particular, the measurement using an automatic immunodiagnostic apparatus is preferred, since it enables the measurement without being affected by endogenous measurement-interference factors and competing enzymes contained in the sample and also enables the quantification of the concentration of AZU1 in the sample in a short period of time.

Another aspect of the second aspect of the present invention is the use of an antibody that specifically recognizes AZU1 in the production of the reagent for detecting cancer (excluding renal cell cancer). Further, it is the use of an antibody that specifically recognizes AZU1 and an antibody or receptor that specifically recognizes any of the second markers listed in Table 2 in the production of the reagent for detecting cancer (excluding renal cell cancer).

Another aspect of the invention is the use of an antibody that specifically recognizes AZU1 in the detection of cancer (excluding renal cell cancer). Furthermore, it is the use of an antibody that specifically recognizes AZU1 and an antibody or receptor that specifically recognizes any of the second markers listed in Table 2 in the detection of cancer (excluding renal cell cancer).

### EXAMPLES

Examples will be shown below to specifically describe the present invention. The following Examples are provided for illustrating the present invention, and the present invention is in no way limited to these Examples.

### [Example 1] Preparation of GPI-Anchor Type AZU1-Expression Plasmid

The region encoding the sequence from isoleucine at the 27th residue to proline at the 248th residue of the amino acid sequence of the human AZU1 protein was amplified by the PCR method. The above-described PCR amplification product was inserted into plasmid pFLAG1 (manufactured by Sigma-Aldrich) containing the coding region of the FLAG tag peptide consisting of the amino acid sequence DYKDDDDK (SEQ ID NO: 1) and the coding region of the signal peptide of the GPI anchor derived from placental alkaline phosphatase using an In-fusion HD cloning kit (manufactured by Takara Bio Inc.), thereby preparing a plasmid capable of expressing GPI-anchor type AZU1, to which the FLAG tag peptide was added to the N-terminal side, and the GPI-anchor type signal peptide was added to the C-terminal side.

### [Example 2] Preparation of Secretory AZU1 Expression Plasmid

The PCR amplification product of the AZU1 coding region prepared in Example 1 was inserted into plasmid pFLAG1 (manufactured by Sigma-Aldrich) containing the coding region of the FLAG tag peptide and the coding region of the BNC peptide (JP 2009-240300 A) consisting of the C-terminal 7-amino acid sequence CKVLRRH (SEQ ID NO: 2) of BNP (brain natriuretic peptide) using an In-fusion HD cloning kit (manufactured by Takara Bio Inc.), thereby preparing a plasmid capable of expressing secretory AZU1, to which the FLAG tag peptide was added to the N-terminal side, and the BNC peptide was added to the C-terminal side.

### [Example 3] Preparation of CHO-K1 Cells Constitutively

### Expressing GPI-Anchor Type AZU1

The GPI-anchor type AZU1 expression plasmid prepared in Example 1 was transfected into a Chinese hamster ovary-derived CHO-K1 cell line according to a conventional method. The cells were then cultured in a 5% CO₂ incubator for 24 hours at 37°C using Ham's F12 medium (manufactured by FUJIFILM Wako Pure Chemical Corporation) supplemented with 10% FBS. After the culture, an antibiotic G418 solution (manufactured by Thermo Fisher Scientific) was added to 250 µg/mL, and the cells were further cultured for three weeks. CHO-K1 cells constitutively expressing GPI-anchor type AZU1 were acquired by a cell sorter using an anti-FLAG antibody.

### [Example 4] Preparation of 293T Cells Transiently Expressing Secretory AZU1

The secretory AZU1 expression plasmid prepared in Example 2 was transfected into a human embryonic kidney-derived 293T cell line according to a conventional method. The cells were then cultured in a 5% CO₂ incubator at 37°C using D-MEM medium (manufactured by FUJIFILM Wako Pure Chemical Corporation) supplemented with 10% FBS such that AZU1 was transiently expressed.

### [Example 5] Collection of Secretory AZU1 Solution

After culturing the 293T cells transiently expressing secretory AZU1 prepared in Example 4 for 72 hours, the culture solution was centrifuged, and the resulting supernatant was collected as a secretory AZU1 solution.

### [Example 6] Collection of Cell-secreted Fine Particles by Ultracentrifugation

The 293T cell line, the 293T cells transiently expressing secretory AZU1 prepared in Example 4, the human renal cancer-derived ACHN cell line, and ACHN cells expressing AZU1-FLAG (Patent Document 4 and Non-patent Document 6) were cultured in a 5% CO₂ incubator using D-MEM medium supplemented with 10% FBS ultrafiltered with AMICON ULTRA-15 - 100 KDa cutoff (manufactured by Merck Millipore) at 37°C for 48 hours. Then the cell-secreted fine particles were collected by the following method.

[1] The culture solution in an amount of 60 mL was centrifuged at 2000 × g at 4°C for 30 minutes, and the resulting supernatant was collected.
[2] The centrifugal supernatant was centrifuged at 16000 × g at 4°C for 30 minutes, and the resulting supernatant was collected.
[3] The centrifugal supernatant was ultracentrifuged at 100000 × g at 4°C for 16 hours, and then the resulting supernatant was removed, and the precipitate was collected.
[4] PBS was added to the ultracentrifugal precipitate, the mixture was centrifuged at 100000 × g at 4°C for 16 hours, and then the resulting supernatant was removed, and the precipitate was collected.
[5] PBS was added in an amount of 200 µL to the ultracentrifugal precipitate, the mixture was suspended by pipetting, and then cell-secreted fine particles were collected.

### [Example 7] Immunization and Cell Fusion

The GPI-anchor type AZU1 expression plasmid constructed in Example 1 was administered at 40 µg/animal to four Balb/c mice every seven days for a total of six times, and then their spleen cells were collected. According to a conventional method, the collected spleen cells and the mouse myeloma Sp2/0 cell line were fused in the presence of polyethylene glycol and cultured in GIT medium (manufactured by FUJIFILM Wako Pure Chemical Corporation) supplemented with HAT (manufactured by Sigma-Aldrich) for about 10 days. Thus, hybridoma cells were selected.

### [Example 8] Screening of Anti-AZUl Antibody-Producing Cells (1)

Hybridoma cells that produce an anti-AZUl antibody were screened by cell enzyme-linked immunosorbent assay (CELISA) described below using the CHO-K1 cells constitutively expressing GPI-anchor type AZU1 prepared in Example 3.

[1] CHO-K1 cells constitutively expressing GPI-anchor type AZU1 were added to a 96-well microplate (manufactured by Falcon) at 5 × 10⁴ cells/well. The cells were then cultured in a 5% CO₂ incubator for 24 hours at 37°C using Ham's F12 medium (manufactured by FUJIFILM Wako Pure Chemical Corporation) supplemented with 10% FBS.
[2] The plate was washed three times with PBS. Mouse IgG diluted to 2 µg/mL with PBS containing 1% BSA (negative control, NC), a commercially available mouse anti-AZUl antibody (manufactured by R&D Systems) (positive control, PC), and the undiluted hybridoma cell culture supernatant were added to the plate at 50 µL/well. The plate was then left to stand at room temperature for one hour.
[3] The plate was washed three times with PBS. A horseradish peroxidase (HRP)-labeled anti-mouse immunoglobulin G-Fc antibody (manufactured by Sigma-Aldrich), which was diluted 20000 times with PBS containing 1% BSA, was added to the plate at 50 µL/well. The plate was then left to stand at room temperature for one hour.
[4] The plate was washed three times with PBS. TMB Microwell Peroxidase Substrate (manufactured by SeraCare Life Sciences) was added to the plate at 50 µL/well. The plate was then left to stand at room temperature for ten minutes.
[5] A 1 mol/L phosphoric acid aqueous solution was added at 50 µL/well to stop the reaction.
[6] The absorbance at 450 nm was measured using an absorbance plate reader.

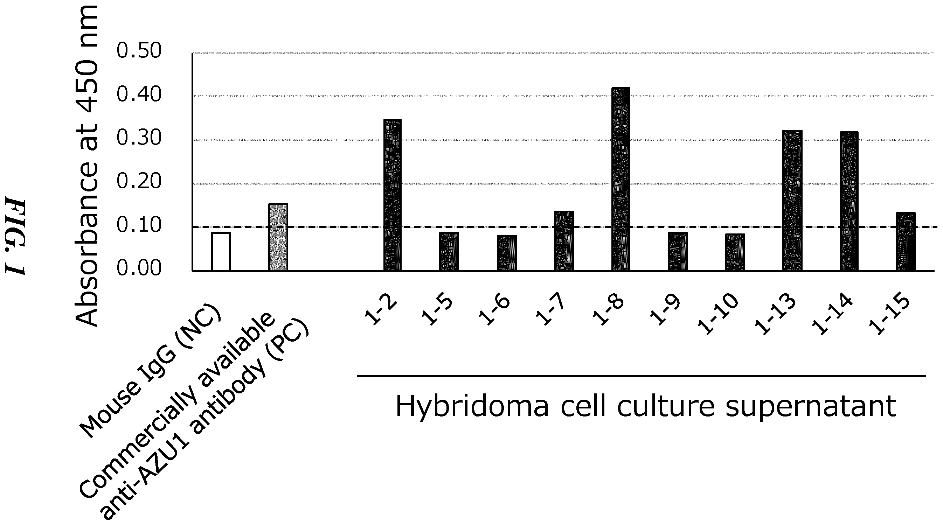

FIG. 1 shows the measurement results. High signals were detected in six hybridoma cell culture supernatants (1-2, 1-7, 1-8, 1-13, 1-14, and 1-15).

### [Example 9] Screening of Anti-AZUl Antibody-Producing Cells (2)

Hybridomas that produce an anti-AZUl antibody were screened by sandwich ELISA described below using the secretory AZU1 solution prepared in Example 5.

[1] An anti-FLAG antibody, which was diluted with carbonate buffer (pH 9.8) to 2 µg/mL, was added at 50 µL/well to a Maxisorp 96-well microplate (manufactured by Thermo Fisher Scientific), and the mixture was allowed to stand overnight at 4°C to be immobilized.
[2] The plate was washed three times with TBS containing 0.05% Tween 20 (TBST). SuperBlock (PBS) (manufactured by Thermo Fisher Scientific) was added to the plate at 200 µL/well. The plate was then left to stand at room temperature for one hour.
[3] The plate was washed three times with TBST. The secretory AZU1 prepared in Example 5, which was diluted 5-fold with PBS containing 1% BSA, was added to the plate at 50 µL/well. The plate was then left to stand at room temperature for two hours.
[4] The plate was washed three times with TBST. Mouse IgG diluted to 2 µg/mL with PBS containing 1% BSA (negative control, NC), a commercially available mouse anti-AZUl antibody (manufactured by R&D Systems) (positive control, PC), and the undiluted hybridoma cell culture supernatant were added to the plate at 50 µL/well. The plate was then left to stand at room temperature for one hour.
[5] The plate was washed three times with TBST. A horseradish peroxidase (HRP)-labeled anti-mouse immunoglobulin G-Fc antibody (manufactured by Sigma-Aldrich), which was diluted 20000 times with PBS containing 1% BSA, was added to the plate at 50 µL/well. The plate was then left to stand at room temperature for one hour.
[6] The plate was washed three times with TBST. SureBlue Reserve TMB (manufactured by SeraCare Life Sciences) was added to the plate at 50 µL/well. The plate was then left to stand at room temperature for ten minutes.
[7] A 1 mol/L phosphoric acid aqueous solution was added at 50 µL/well to stop the reaction.
[8] The absorbance at 450 nm was measured using an absorbance plate reader.

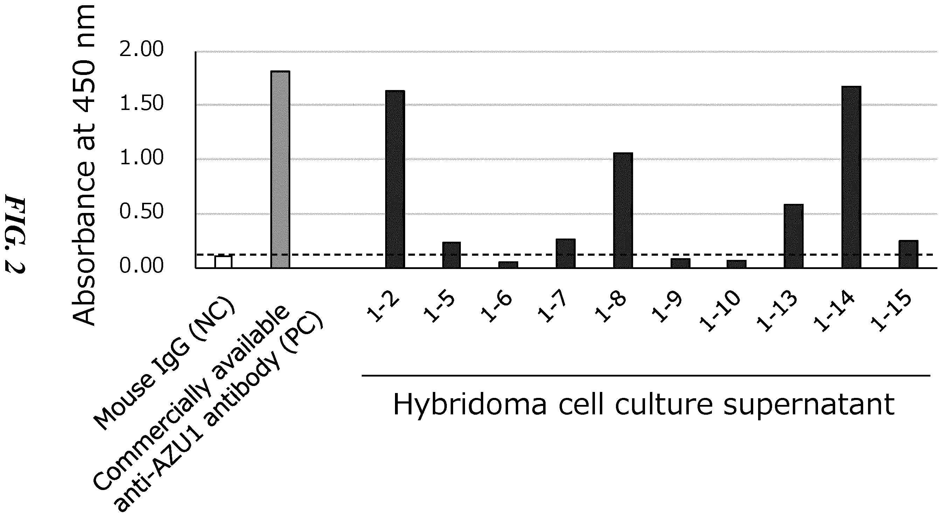

FIG. 2 shows the measurement results. High signals were detected in seven hybridoma cell culture supernatants (1-2, 1-5, 1-7, 1-8, 1-13, 1-14, and 1-15).

### [Example 10] Cloning of Anti-AZUl Antibody-Producing Cells

Cloning of three hybridoma cells (1-2, 1-8, and 1-14) selected based on the results of Example 8 and Example 9 was performed by the limiting dilution method. The resulting clones were cultured in GIT medium supplemented with HT (manufactured by Sigma-Aldrich) and then adapted to HT-free GIT medium. Finally, three anti-AZUl antibody-producing cell lines (clones 1-2, 1-8, and 1-14) were established.

### [Example 11] Preparation of Anti-AZUl Monoclonal Antibody

Monoclonal antibodies were purified from the culture supernatants of the three anti-AZUl antibody-producing cells (clones 1-2, 1-8, and 1-14) established in Example 10 using a Protein G column according to a conventional method. After dialysis of each purified antibody with PBS, the absorbance at 280 nm was measured so as to quantify the protein concentration.

### [Example 12] Preparation of Biotin-labeled Antibody

An anti-CD81 antibody, an anti-CD9 antibody, and an anti-CD63 antibody (all manufactured by Frontier Institute), and three anti-AZUl antibodies prepared in Example 11 (clones 1-2, 1-8, and 1-14) were biotin-labeled using a Biotin labeling Kit-NH₂ labeling kit (manufactured by Dojindo Laboratories) according to the protocol described in the product instruction manual.

### [Example 13] Performance Evaluation of Anti-AZUl Monoclonal Antibody (1)

Secretory fine particles derived from 293T, 293T-AZU1, ACHN, and ACHN-AZU1 cells prepared in Example 6 were measured by sandwich ELISA with eight combinations listed in Table 3.

### [Table 3]

**(Table 3)**

| **Condition** | **Solid-phase antibody** | **Biotin-labeled antibody** |
|---|---|---|
| 1 | Commercially available anti-AZUl antibody | Commercially available anti-CD63 antibody |
| 2 | Anti-AZUl antibody clone 1-2 | Commercially available anti-CD63 antibody |
| 3 | Anti-AZUl antibody clone 1-8 | Commercially available anti-CD63 antibody |
| 4 | Anti-AZU1 antibody clone 1-14 | Commercially available anti-CD63 antibody |
| 5 | Commercially available anti-CD63 antibody | Commercially available anti-AZU1 antibody |
| 6 | Commercially available anti-CD63 antibody | Anti-AZUl antibody clone 1-2 |
| 7 | Commercially available anti-CD63 antibody | Anti-AZUl antibody clone 1-8 |
| 8 | Commercially available anti-CD63 antibody | Anti-AZU1 antibody clone 1-14 |

The specific method is described below.
[1] A solid-phase antibody listed in Table 3, which was diluted with carbonate buffer (pH 9.8) to 4 µg/mL, was added at 50 µL/well to a Maxisorp 96-well microplate (manufactured by Thermo Fisher Scientific), and the mixture was allowed to stand overnight at 4°C to be immobilized.
[2] The plate was washed three times with PBS. SuperBlock (PBS) (manufactured by Thermo Fisher Scientific) was added to the plate at 300 µL/well. The plate was then left to stand at room temperature for one hour.
[3] The plate was washed three times with PBS. The ultracentrifugal precipitate, which was diluted 10-fold with PBS containing 1% BSA, was added to the plate at 50 µL/well. The plate was then left to stand at room temperature for two hours.
[4] The plate was washed three times with PBS. A biotin-labeled antibody listed in Table 3, which was diluted to 2 µg/mL with PBS containing 1% BSA, was added to the plate at 50 µL/well. The plate was then left to stand at room temperature for one hour.
[5] The plate was washed three times with PBS. Streptavidin-PolyHRP40 (manufactured by Stereospecific Detection technologies), which was diluted 50000-fold with PBS containing 1% BSA, was added to the plate at 50 µL/well. The plate was then left to stand at room temperature for 30 minutes.
[6] The plate was washed three times with PBS. SureBlue Reserve TMB (manufactured by SeraCare Life Sciences) was added to the plate at 50 µL/well. The plate was then left to stand at room temperature for ten minutes.
[7] A 1 mol/L phosphoric acid aqueous solution was added at 50 µL/well to stop the reaction.
[8] The absorbance at 450 nm was measured using an absorbance plate reader.

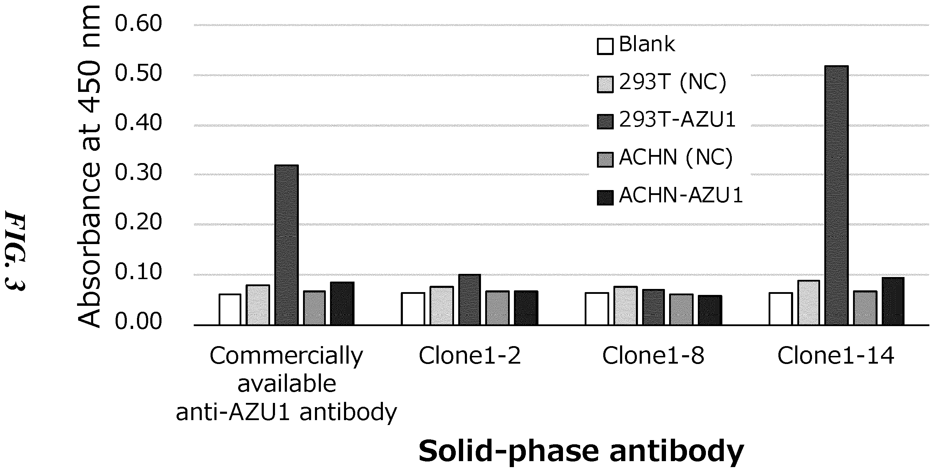

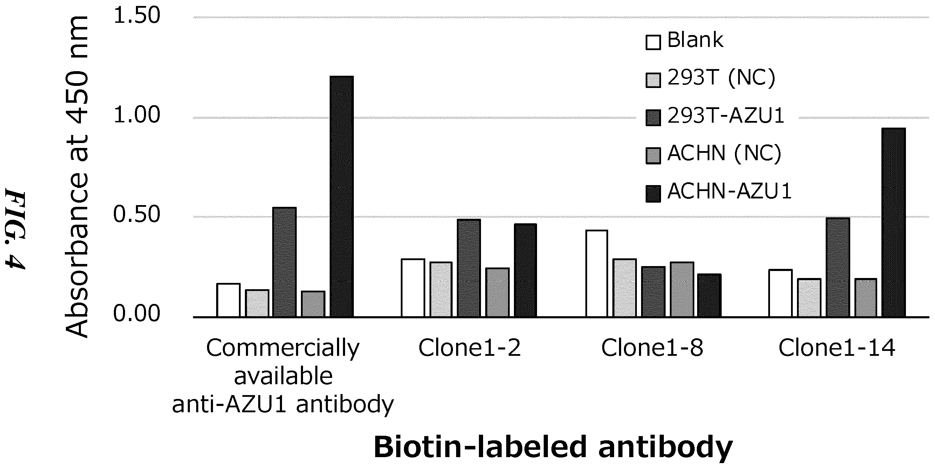

FIGS. 3 and 4 show the measurement results (NC stands for negative control). It was shown that cell-secreted fine particles containing AZU1 can be detected by using an anti-AZUl antibody as either a solid-phase antibody or biotin-labeled antibody. In particular, clone 1-14 was shown to have high detection sensitivity.

Hereinafter, an anti-AZUl antibody will be referred to as clone 1-14 unless otherwise specified.

### [Example 14] Performance Evaluation of Anti-AZUl Monoclonal Antibody (2)

Secretory fine particles derived from 293T, 293T-AZU1, ACHN, and ACHN-AZU1 cells prepared in Example 6 were measured by sandwich ELISA with three combinations listed in Table 4.

[Table 4]

**(Table 4)**

| **Condition** | **Solid-phase antibody** | **Blotin-labeled antibody** |
|---|---|---|
| 1 | Commercially available anti-CD81 antibody | Anti-AZUl antibody clone 1-14 |
| 2 | Commercially available anti-CD9 antibody | Anti-AZUl antibody clone 1-14 |
| 3 | Commercially available anti-CD63 antibody | Anti-AZU1 antibody clone 1-14 |

The specific method is the same as in Example 13.

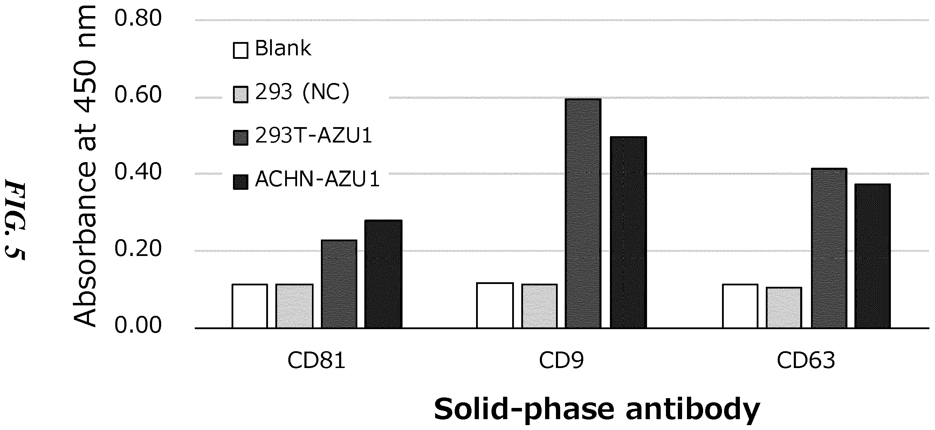

FIG. 5 shows the measurement results (NC stands for negative control). It was shown that cell-secreted fine particles containing AZU1 can be detected by using any one of an anti-CD81 antibody, an anti-CD9 antibody, and an anti-CD63 antibody as the solid-phase antibody.

### [Example 15] Measurement of AZU1 in Serum Samples from Healthy Individuals and Stomach Cancer Patients (1)

The details of the serum samples from healthy individuals used in this Example are shown in Table 5. All sera from healthy individuals were collected at the Health Service Center of the Tokyo Research Center of Tosoh Corporation with the approval of the ethics committee of the Bioscience Division of Tosoh Corporation and the consent of the sample providers.

[Table 5]

**(Table 5)**

| **Sample ID** | **Age** | **Sex** |
|---|---|---|
| N1 | 56 | Male |
| N2 | 59 | Male |
| N3 | 56 | Male |
| N4 | 58 | Male |
| N5 | 58 | Male |
| N6 | 62 | Male |
| N7 | 56 | Male |

The details of the serum samples of stomach cancer patients used in this Example are shown in Table 6. The serum samples of stomach cancer patients were purchased from ProMedDx, LLC, and BioIVT. It is clearly described in the documents attached to the products of both companies that these samples were collected in accordance with the protocols approved by the ethics committee.

[Table 6]

**(Table 6)**

| **Sample ID** | **Age** | **Sex** | **Stomach cancer stage** |
|---|---|---|---|
| S01 | 55 | Male | IV |
| S03 | 70 | Male | IV |
| S04 | 69 | Male | IIB/III |
| S05 | 72 | Male | IV |
| S06 | 65 | Male | IV |
| S07 | 65 | Male | IA |
| S10 | 85 | Male | IV |
| S14 | 79 | Male | IV |
| S15 | 62 | Male | II |
| S17 | 54 | Male | III |

The above-described serum samples were measured by sandwich ELISA with six combinations listed in Table 7.

[Table 7]

**(Table 7)**

| **Condition** | **Solid-phase antibody** | **Biotin-labeled antibody** |
|---|---|---|
| 1 | Commercially available anti-CD81 antibody | Anti-AZU1 antibody clone 1-14 |
| 2 | Commercially available anti-CD9 antibody | Anti-AZUl antibody clone 1-14 |
| 3 | Commercially available anti-CD63 antibody | Anti-AZUl antibody clone 1-14 |
| 4 | Anti-AZU1 antibody clone 1-14 | Commercially available anti-CD81 antibody |
| 5 | Anti-AZU1 antibody clone 1-14 | Commercially available anti-CD9 antibody |
| 6 | Anti-AZUl antibody clone 1-14 | Commercially available anti-CD63 antibody |

The specific method is described below.
[1] A solid-phase antibody listed in Table 7, which was diluted with carbonate buffer (pH 9.8) to 4 µg/mL, was added at 50 µL/well to a Maxisorp 96-well microplate (manufactured by Thermo Fisher Scientific), and the mixture was allowed to stand overnight at 4°C to be immobilized.
[2] The plate was washed three times with PBS. SuperBlock (PBS) (manufactured by Thermo Fisher Scientific) was added to the plate at 300 µL/well. The plate was then left to stand at room temperature for one hour.
[3] The plate was washed three times with PBS. A serum sample which was diluted 5-fold with PBS containing 1% BSA and a 0.05 mg/mL heterophilic blocking reagent (HBR1) (manufactured by Scantibodies Laboratory), was added to the plate at 50 µL/well. The plate was then left to stand at room temperature for two hours.
[4] The plate was washed three times with PBS. A biotin-labeled detection antibody listed in Table 7, which was diluted to 2 µg/mL with PBS containing 1% BSA, was added to the plate at 50 µL/well. The plate was then left to stand at room temperature for one hour.
[5] The plate was washed three times with PBS. Streptavidin-PolyHRP40 (manufactured by Stereospecific Detection technologies), which was diluted 50000-fold with PBS containing 1% BSA, was added to the plate at 50 µL/well. The plate was then left to stand at room temperature for one hour.
[6] The plate was washed three times with PBS. SureBlue Reserve TMB (manufactured by SeraCare Life Sciences) was added to the plate at 50 µL/well. The plate was then left to stand at room temperature for ten minutes.
[7] A 1 mol/L phosphoric acid aqueous solution was added at 50 µL/well to stop the reaction.
[8] The absorbance at 450 nm was measured using an absorbance plate reader.

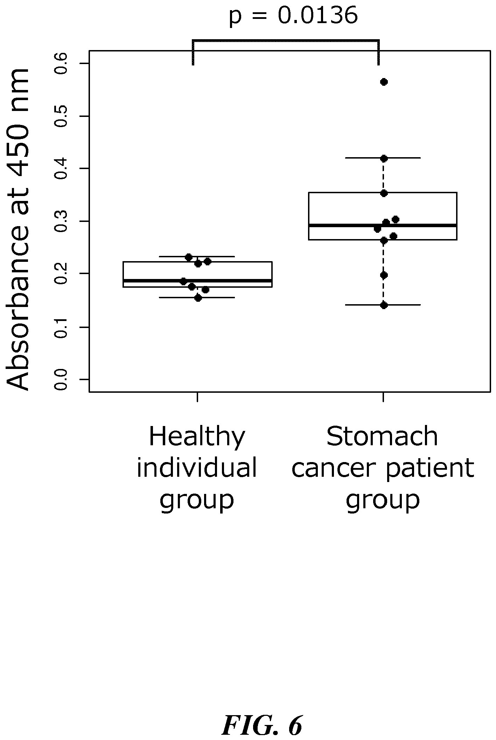

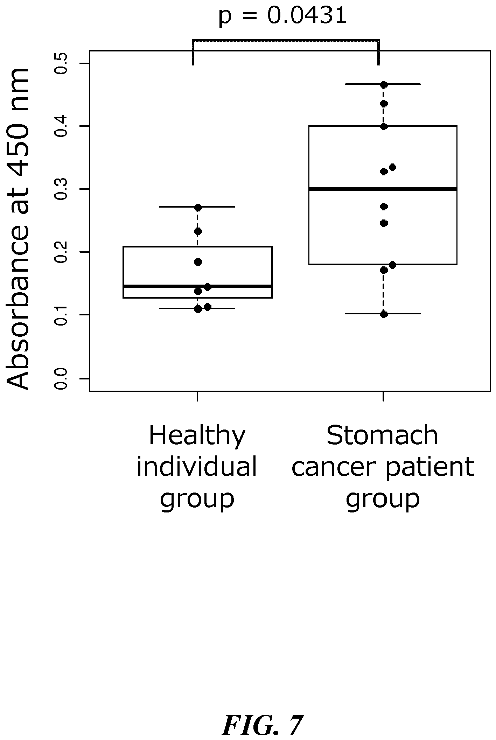

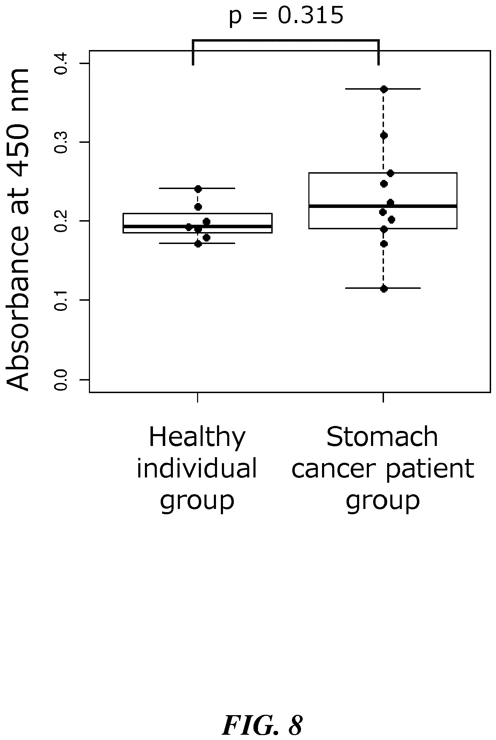

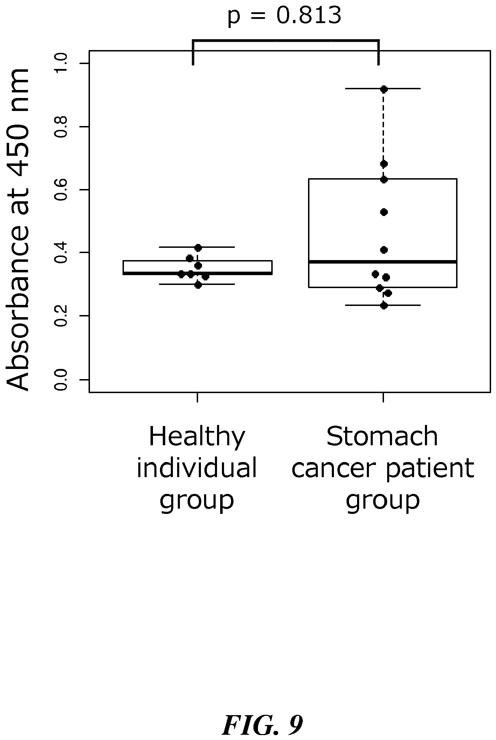

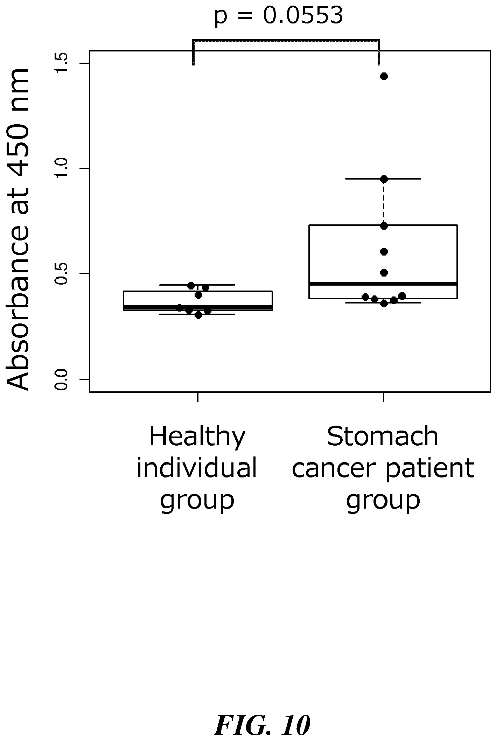

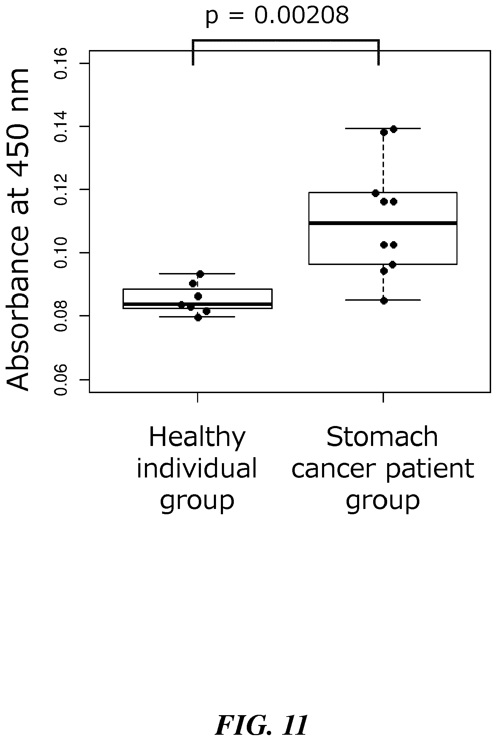

FIGS. 6 to 11 show box plots of absorbance in sandwich ELISA performed with six combinations. The minimum value, 25th percentile, median, 75th percentile, and maximum value of absorbance, and range of absorbance in the 95% confidence interval of each of the healthy individual group and the stomach cancer patient group in sandwich ELISA performed with six combinations, and the p-values of the Mann-Whitney U test are shown in Table 8. In all cases of sandwich ELISA, the values in the stomach cancer patient group tended to be higher than those in the healthy individual group. In particular, in the cases of using the combinations of the solid phase antibody and the biotin-labeled antibody in Examples 15-1, 15-2, and 15-6, a statistically significant difference was confirmed (p < 0.05).

### [Table 8]

**(Table 8)**

| **Example** | 15-1 | | 15-2 | | 15-3 | | 15-4 | | 15-5 | | 15-6 | |
|---|---|---|---|---|---|---|---|---|---|---|---|---|
| **Solid-phase antibody** | Anti-CD81 antibody | | Anti-CD9 antibody | | Anti-CD63 antibody | | Anti-AZU1 antibody | | Anti-AZUl antibody | | Anti-AZUl antibody | |
| **Biotin-labeled antibody** | Anti-AZUl antibody | | Anti-AZUl antibody | | Anti-AZUl antibody | | Anti-CD81 antibody | | Anti-CD9 antibody | | Anti-CD63 antibody | |
| **Group** | Healthy | Stomach cancer | Healthy | Stomach cancer | Healthy | Stomach cancer | Healthy | Stomach cancer | Healthy | Stomach cancer | Healthy | Stomach cancer |
| **Minimum** | 0.1555 | 0.1417 | 0.1103 | 0.1028 | 0.1723 | 0.1156 | 0.3012 | 0.2364 | 0.3056 | 0.3639 | 0.0799 | 0.0853 |
| **25th Percentile** | 0.1745 | 0.2665 | 0.1267 | 0.1976 | 0.1858 | 0.1937 | 0.3316 | 0.2985 | 0.3296 | 0.3853 | 0.0826 | 0.0981 |
| **Median** | 0.1875 | 0.2926 | 0.1454 | 0.3012 | 0.1942 | 0.2188 | 0.3365 | 0.3723 | 0.3440 | 0.4530 | 0.0837 | 0.1096 |
| **75th Percentile** | 0.2223 | 0.3414 | 0.2093 | 0.3843 | 0.2103 | 0.2586 | 0.3736 | 0.6096 | 0.4193 | 0.7022 | 0.0886 | 0.1184 |
| **Maximum** | 0.2323 | 0.5671 | 0.2726 | 0.4673 | 0.2414 | 0.3686 | 0.4174 | 0.9210 | 0.4478 | 1.4436 | 0.0936 | 0.1395 |
| **95% Confidence interval** | 0.1734- | 0.2376- | 0.1256- | 0.2198- | 0.1824- | 0.1864- | 0.3233- | 0.326- | 0.3282- | 0.3993- | 0.082- | 0.0999- |
| | 0.2177 | 0.3838 | 0.2174 | 0.3695 | 0.2176 | 0.2750 | 0.3811 | 0.6026 | 0.4133 | 0.8322 | 0.0893 | 0.1225 |
| **P value** | 0.0136 | | 0.0431 | | 0.315 | | 0.813 | | 0.0553 | | 0.00208 | |

### [Example 16] Measurement of AZU1 in Serum Samples from Healthy Individuals and Stomach Cancer Patients (2)

The same serum samples used in Example 15 were measured by sandwich ELISA described below.
[1] Phosphatidylserine receptor Tim4-hFc (manufactured by FUJIFILM Wako Pure Chemical Corporation), which was diluted to 4 µg/mL with PBS, was added at 50 µL/well to a Maxisorp 96-well microplate (manufactured by Thermo Fisher Scientific), and the mixture was allowed to stand overnight at 4°C to be immobilized.
[2] The plate was washed three times with PBS. SuperBlock (PBS) (manufactured by Thermo Fisher Scientific) was added to the plate at 300 µL/well. The plate was then left to stand at room temperature for one hour.
[3] The plate was washed three times with TBS. A serum sample which was diluted 5-fold with TBS containing 1% BSA, 2 mM CaCl₂, and a 0.05 mg/mL heterophilic blocking reagent (HBR1) (manufactured by Scantibodies Laboratory) was added to the plate at 50 µL/well. The plate was then left to stand at room temperature for two hours.
[4] The plate was washed three times with TBS containing 2 mM CaCl₂. A biotin-labeled anti-AZUl antibody (clone 1-14), which was diluted to 2 µg/mL with TBS containing 1% BSA and 2 mM CaCl₂, was added to the plate at 50 µL/well. The plate was then left to stand at room temperature for one hour.
[5] The plate was washed three times with TBS containing 2 mM CaCl₂. Streptavidin-PolyHRP40 (manufactured by Stereospecific Detection technologies), which was diluted 50000-fold with TBS containing 1% BSA and 2 mM CaCl₂, was added to the plate at 50 µL/well. The plate was then left to stand at room temperature for one hour.
[6] The plate was washed three times with TBS containing 2 mM CaCl₂. SureBlue Reserve TMB (manufactured by SeraCare Life Sciences) was added to the plate at 50 µL/well. The plate was then left to stand at room temperature for ten minutes.
[7] A 1 mol/L phosphoric acid aqueous solution was added at 50 µL/well to stop the reaction.
[8] The absorbance at 450 nm was measured using an absorbance plate reader.

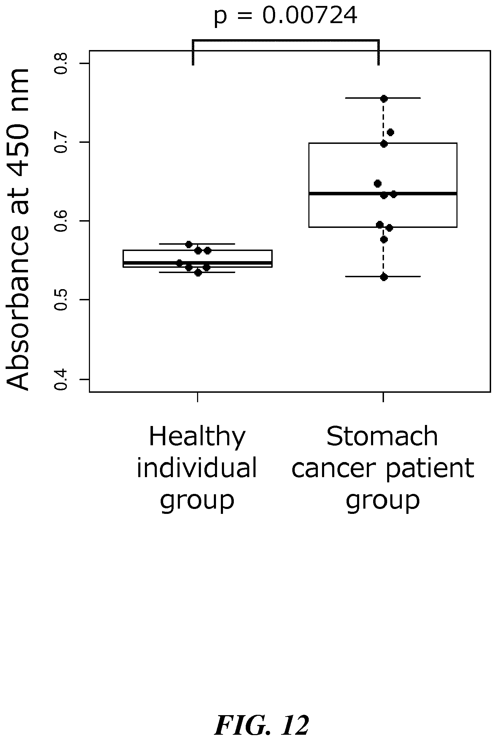

FIG. 12 shows box plots of absorbance. The minimum value, 25th percentile, median, 75th percentile, and maximum value of absorbance, and range of absorbance in the 95% confidence interval of each of the healthy individual group and the stomach cancer patient group, and the p-value of the Mann-Whitney U test are shown in Table 9. In the stomach cancer patient group, the values tended to be higher than those in the healthy individual group, showing a statistically significant difference (p < 0.05).

### [Table 9]

**(Table 9)**

| **Example** | 16 | |
|---|---|---|
| **Solid-phase receptor Biotin-labeled antibody** | Tim4-hFc Anti-AZU1 antibody | |
| **Group** | Healthy | Stomach cancer |
| **Minimum** | 0.5351 | 0.5297 |
| **25th Percentile** | 0.5420 | 0.5937 |
| **Median** | 0.5467 | 0.6346 |
| **75th Percentile** | 0.5627 | 0.6865 |
| **Maximum** | 0.5707 | 0.7569 |
| **95% Confidence interval** | 0.5417- | 0.5957- |
| | 0.5617 | 0.6811 |
| **P value** | 0.00724 | |

### [Comparative Example 1] Measurement of CEA in Serum Samples from Healthy Individuals and Stomach Cancer Patients

CEA, which is an existing representative stomach cancer marker, was measured for the same serum samples used in Examples 15 and 16 using a fully automatic enzyme immunoassay apparatus, AIA-2000 (manufactured by Tosoh Corporation), as an apparatus for evaluation and a carcinoembryonic antigen (CEA) measurement reagent (manufactured by Tosoh Corporation, Approval No. 20100EZZ00112000).

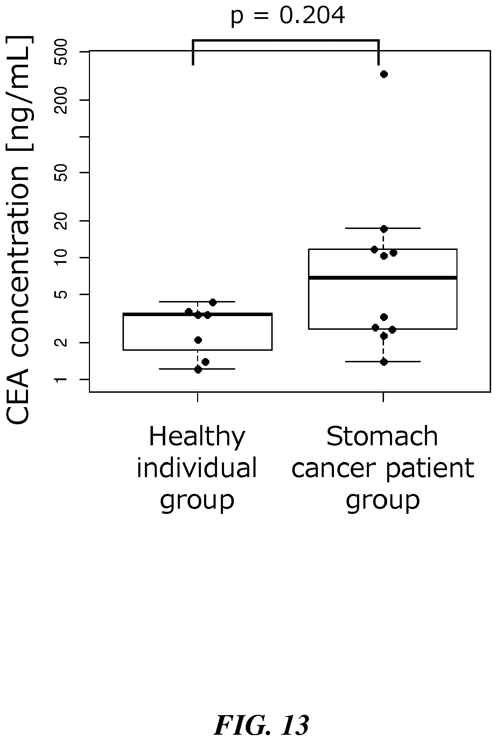

FIG. 13 shows box plots of measured values. The minimum absorbance, 25th percentile, median, 75th percentile, maximum value, and range of absorbance in the 95% confidence interval of each of the healthy individual group and the stomach cancer patient group, and the p-value of the Mann-Whitney U test are shown in Table 10.

### [Table 10]

**(Table 10)**

| | | |
|---|---|---|
| **Comparative Example** | 1 | |
| **Measurement Item** | CEA [ng/mL] | |

| **Group** | Healthy | Stomach cancer |
|---|---|---|
| **Minimum** | 1.2 | 1.4 |
| **25th Percentile** | 1.8 | 2.6 |
| **Median** | 3.4 | 6.9 |
| **75th Percentile** | 3.5 | 11.6 |
| **Maximum** | 4.3 | 326.8 |
| **95% Confidence interval** | 1.9-3.7 | -23.8-101.8 |
| **P value** | 0.204 | |

### [Example 17] Comparison of Stomach Cancer Detection Performance between AZU1 and CEA

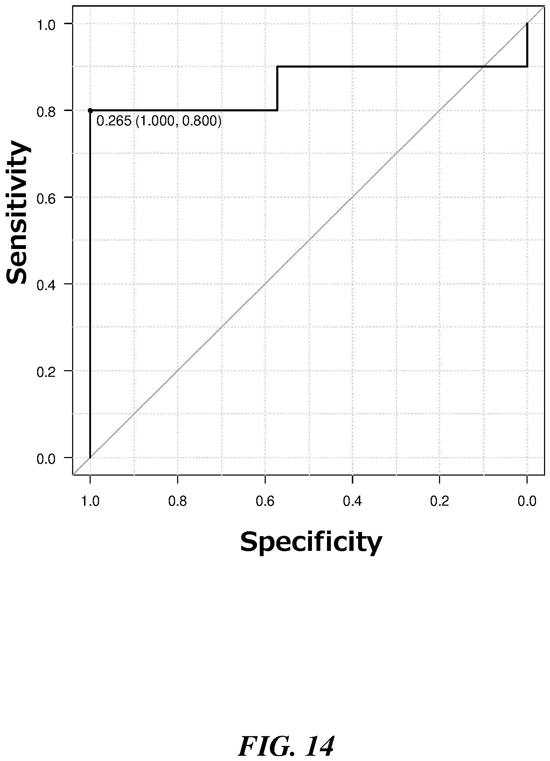

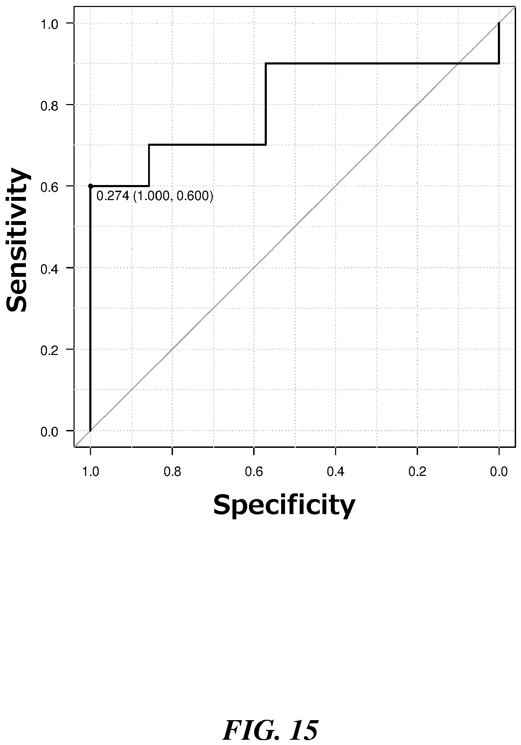

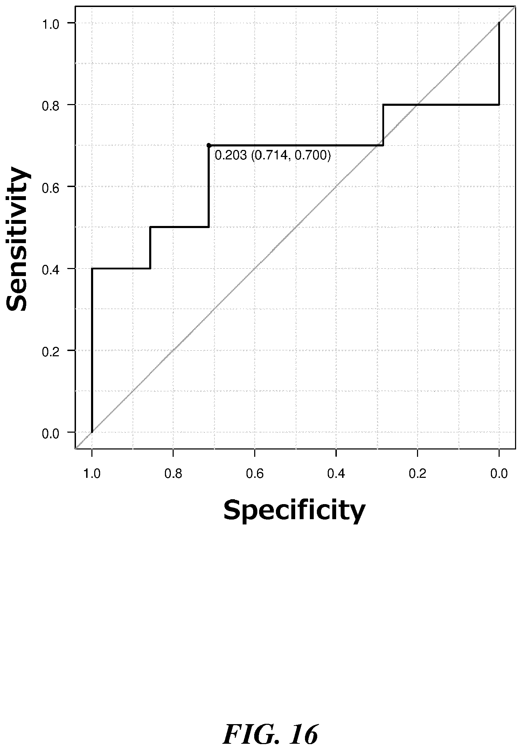

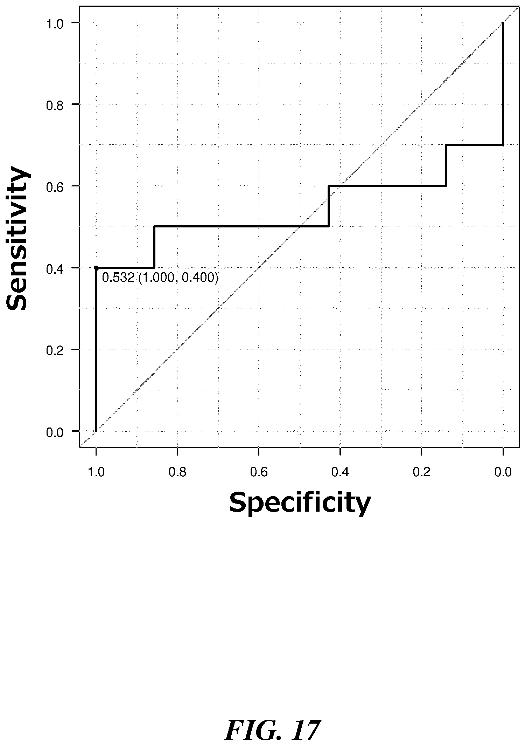

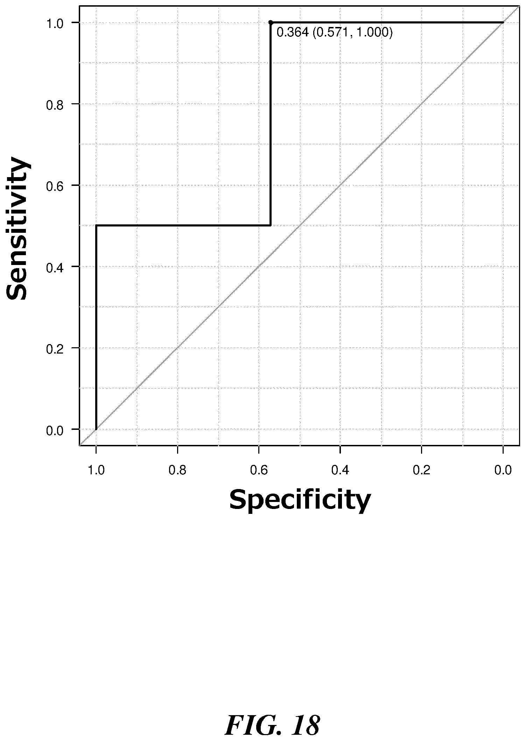

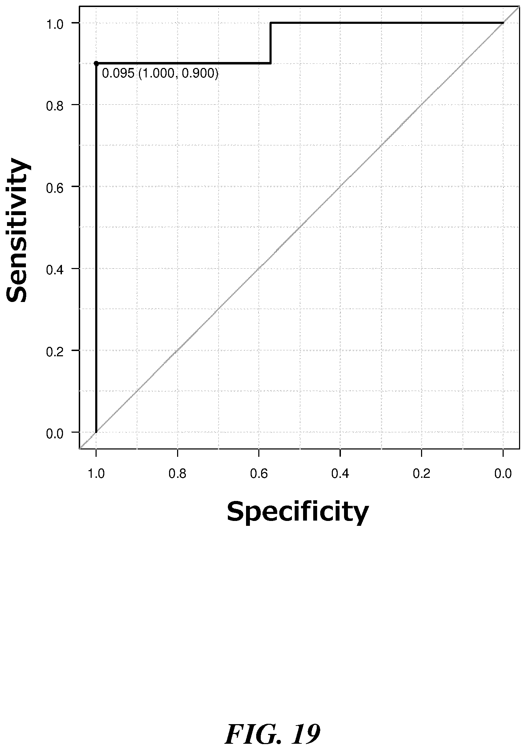

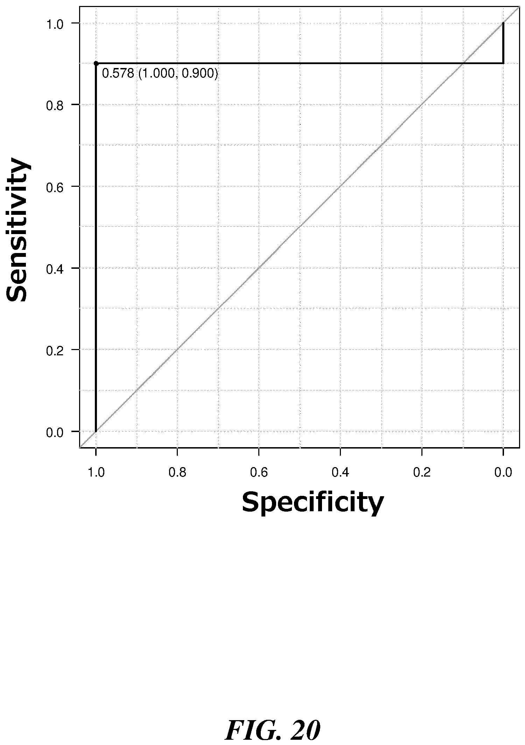

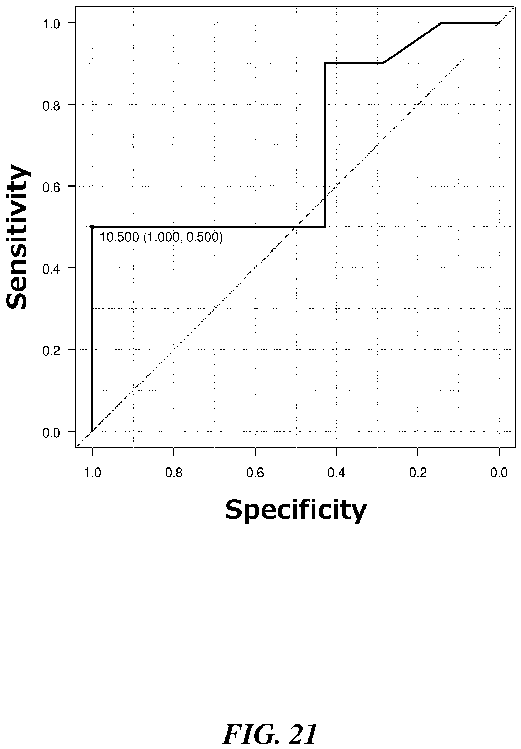

Based on the measurement results of Examples 15 to 16 and Comparative Example 1, FIGS. 14 to 21 show the results of receiver operating characteristic (ROC) curve analysis for discrimination between the healthy individual group and the stomach cancer patient group, and Table 11 shows the area under the ROC curve (AUC) and the range of AUC in the 95% confidence interval. The AUC of AZU1 was higher than that of CEA in Examples 15-1, 15-2, 15-5, 15-6, and 16, indicating that AZU1 has excellent stomach cancer detection performance.

[Table 11]

**(Table 11)**

| **Example** | **Solid-phase antibody or the like** | **Biotin-labeled antibody** | **AUC** | **95% Confidence interval** |
|---|---|---|---|---|
| 15-1 | Anti-CD81 antibody | Anti-AZUl antibody | 0.857 | 0.649 - 1 |
| 15-2 | Anti-CD9 antibody | Anti-AZUl antibody | 0.800 | 0.573 - 1 |
| 15-3 | Anti-CD63 antibody | Anti-AZUl antibody | 0.657 | 0.379 - 0.935 |
| 15-4 | Anti-AZUl antibody | Anti-CD81 antibody | 0.543 | 0.242 - 0.843 |
| 15-5 | Anti-AZUl antibody | Anti-CD9 antibody | 0.786 | 0.543 - 1 |
| 15-6 | Anti-AZUl antibody | Anti-CD63 antibody | 0.957 | 0.864 - 1 |
| 16 | Tim4-hFc | Anti-AZUl antibody | 0.900 | 0.704 - 1 |
| Comparative Example 1 | CEA measurement reagent | | 0.693 | 0.421 - 0.965 |

Table 12 shows the sensitivity and specificity in a case where the value that maximizes the Youden's index calculated by sensitivity + specificity - 1 in ROC curve analysis was set as the cutoff value for Examples 15 and 16 and in a case where a general CEA reference value of 5.0 ng/mL was set as the cutoff value for Comparative Example 1. AZU1 had higher sensitivity than and comparable specificity to CEA in Examples 15-1, 15-2, 15-6, and 16, indicating that AZU1 has excellent stomach cancer detection performance.

[Table 12]

**(Table 12)**

| **Example** | **Solid-phase antibody or the like** | **Biotin-labeled antibody** | **Cut-off value** | **Sensitivity** | **Specificity** |
|---|---|---|---|---|---|
| 15-1 | Anti-CD81 antibody | Anti-AZUl antibody | 0.265 | 0.70 | 1.00 |
| 15-2 | Anti-CD9 antibody | Anti-AZU1 antibody | 0.274 | 0.60 | 1.00 |
| 15-3 | Anti-CD63 antibody | Anti-AZUl antibody | 0.203 | 0.70 | 0.71 |
| 15-4 | Anti-AZU1 antibody | Anti-CD81 antibody | 0.532 | 0.30 | 1.00 |
| 15-5 | Anti-AZU1 antibody | Anti-CD9 antibody | 0.364 | 1.00 | 0.57 |
| 15-6 | Anti-AZU1 antibody | Anti-CD63 antibody | 0.095 | 0.80 | 1.00 |
| 16 | Tim4-hFc | Anti-AZU1 antibody | 0.578 | 0.90 | 1.00 |
| Comparative Example 1 | CEA measurement reagent | | 5.0 ng/mL | 0.50 | 1.00 |

### [Example 18] Measurement of Serum Samples from Breast Cancer Patients

The serum samples of healthy individuals used in this Example are the same as those used in Examples 15 and 16 and Comparative Example 1. The details of the serum samples of breast cancer patients used in this Example are shown in Table 13. The serum samples of breast cancer patients were purchased from ProMedDx, LLC. It is clearly described in the documents attached to the products of the company that these samples were collected in accordance with the protocols approved by the ethics committee.

[Table 13]

**(Table 13)**

| **Sample ID** | **Age** | **Sex** | **Breast cancer stage** |
|---|---|---|---|
| B01 | 66 | Female | 0 |
| B02 | 61 | Female | IIA |
| B03 | 71 | Female | IA |
| B04 | 74 | Female | IIIA |
| B05 | 64 | Female | 0 |
| B06 | 75 | Female | IA |
| B07 | 68 | Female | 0 |
| B08 | 68 | Female | IIIC |
| B09 | 78 | Female | IIIA |
| B10 | 68 | Female | IIA |
| B11 | 62 | Female | IA |

The above-described samples were measured by sandwich ELISA described in Conditions 1 to 3 of Table 7. The specific method is the same as in Example 15.

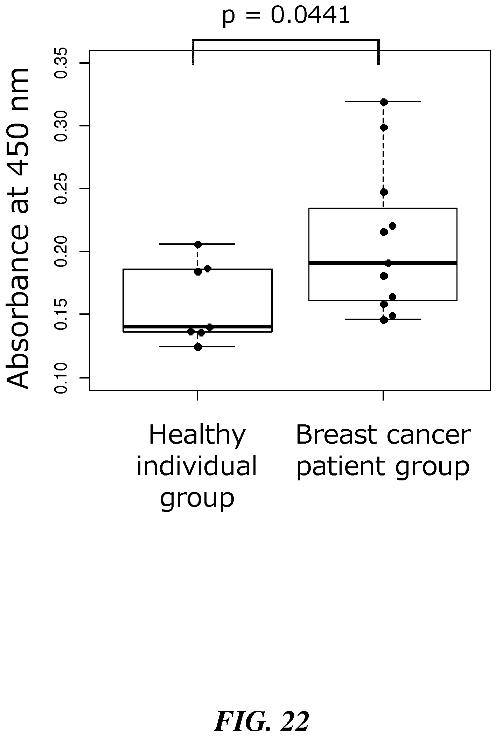

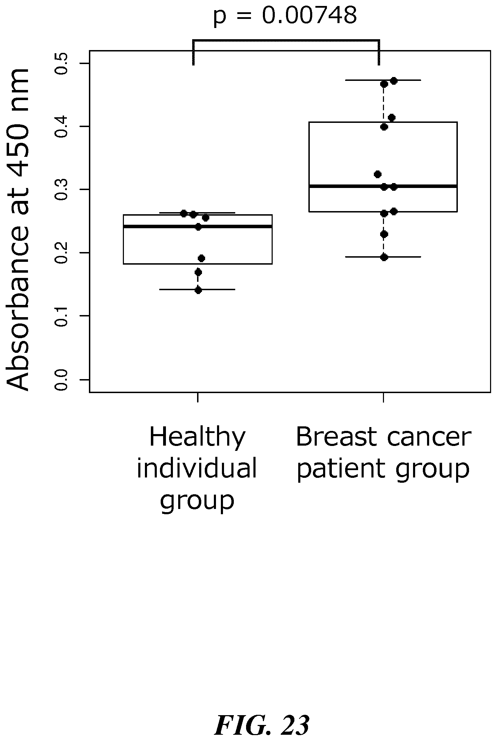

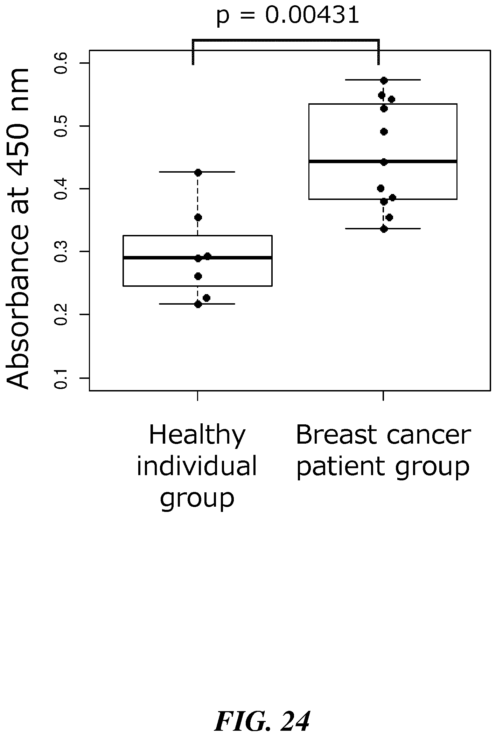

FIGS. 22 to 24 show box plots of absorbance. The minimum value, 25th percentile, median, 75th percentile, and maximum value of absorbance, and range of absorbance in the 95% confidence interval of each of the healthy individual group and the breast cancer patient group, and the p-values of the Mann-Whitney U test are shown in Table 14. In all cases of sandwich ELISA, the values in the breast cancer patient group tended to be higher than those in the healthy individual group, showing a statistically significant difference (p < 0.05).

[Table 14]

**(Table 14)**

| **Example** | 18-1 | | 18-2 | | 18-3 | |
|---|---|---|---|---|---|---|
| **Solid-phase antibody** | Anti-CD81 antibody | | Anti-CD9 antibody | | Anti-CD63 antibody | |
| **Biotin-labeled antibody** | Anti-AZU1 antibody | | Anti-AZUl antibody | | Anti-AZU1 antibody | |
| **Group** | Healty | Breast cancer | Healty | Breast cancer | Healty | Breast cancer |
| **Minimum** | 0.1250 | 0.1463 | 0.1422 | 0.1937 | 0.2174 | 0.3373 |
| **25th Percentile** | 0.1366 | 0.1614 | 0.1824 | 0.2652 | 0.2450 | 0.3834 |
| **Median** | 0.1407 | 0.1911 | 0.2426 | 0.3064 | 0.2913 | 0.4439 |
| **75th Percentile** | 0.1861 | 0.2342 | 0.2600 | 0.4075 | 0.3251 | 0.5356 |
| **Maximum** | 0.2064 | 0.3196 | 0.2635 | 0.4744 | 0.4274 | 0.5736 |
| **95% Confidence interval** | 0.1358-0.1835 | 0.1736-0.2436 | 0.1823-0.2557 | 0.2758-0.3880 | 0.2417-0.3515 | 0.4030-0.5051 |
| **P value** | 0.0441 | | 0.00748 | | 0.00431 | |

### [Example 19] Measurement of Serum Samples from Colorectal Cancer Patients

The serum samples of healthy individuals used in this Example are the same as those used in Examples 15 and 16 and Comparative Example 1. The details of the serum samples of colorectal cancer patients used in this Example are shown in Table 15. The serum samples of colorectal cancer patients were purchased from ProMedDx, LLC. It is clearly described in the documents attached to the products of the company that these samples were collected in accordance with the protocols approved by the ethics committee.

[Table 15]

**(Table 15)**

| **Sample ID** | **Age** | **Sex** | **Colorectal cancer stage** |
|---|---|---|---|
| C03 | 71 | Male | IIA |
| C04 | 63 | Male | I |
| C05 | 65 | Male | IIA |
| C10 | 65 | Male | I |
| C13 | 81 | Male | IVB |
| C15 | 54 | Male | IVA |

The above-described samples were measured by sandwich ELISA described in Conditions 1 to 3 of Table 7. The specific method is the same as in Example 15.

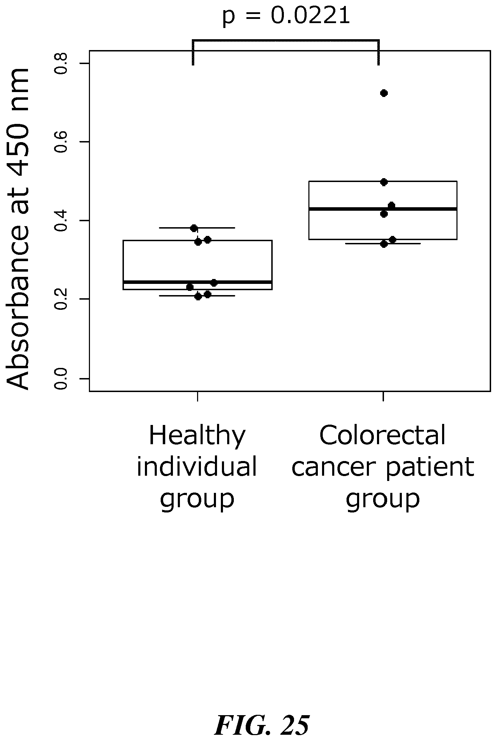

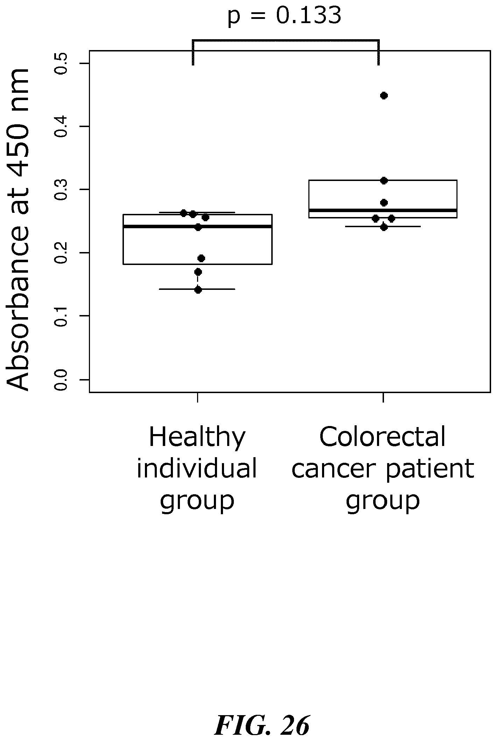

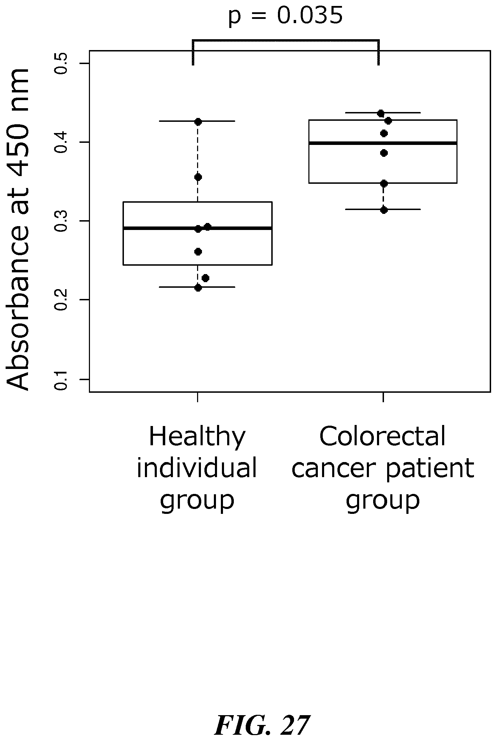

FIGS. 25 to 27 show box plots of absorbance. The minimum value, 25th percentile, median, 75th percentile, and maximum value of absorbance, and range of absorbance in the 95% confidence interval of each of the healthy individual group and the colorectal cancer patient group, and the p-values of the Mann-Whitney U test are shown in Table 16. In all cases of sandwich ELISA, the values in the colorectal cancer patient group tended to be higher than those in the healthy individual group. In particular, in the cases of using the combinations of the solid phase antibody and the biotin-labeled antibody in Examples 20-1 and 20-3, a statistically significant difference was confirmed (p < 0.05).

[Table 16]

**(Table 16)**

| **Example** | 19-1 | | 19-2 | | 19-3 | |
|---|---|---|---|---|---|---|
| **Solid-phase antibody** | Anti-CD81 antibody | | Anti-CD9 antibody | | Anti-CD63 antibody | |
| **Biotin-labeled antibody** | Anti-AZUl antibody | | Anti-AZUl antibody | | Anti-AZUl antibody | |
| **Group** | Healty | Colorectal cancer | Healty | Colorectal cancer | Healty | Colorectal cancer |
| **Minimum** | 0.2105 | 0.3422 | 0.1422 | 0.2424 | 0.2174 | 0.3146 |
| **25th Percentile** | 0.2241 | 0.3697 | 0.1824 | 0.2547 | 0.2450 | 0.3576 |
| **Median** | 0.2446 | 0.4293 | 0.2426 | 0.2683 | 0.2913 | 0.3994 |
| **75th Percentile** | 0.3513 | 0.4850 | 0.2600 | 0.3071 | 0.3251 | 0.4239 |
| **Maximum** | 0.3829 | 0.7248 | 0.2635 | 0.4509 | 0.4274 | 0.4376 |
| **95% Confidence interval** | 0.2291-0.3392 | 0.3506-0.5758 | 0.1823-0.2557 | 0.2372-0.3628 | 0.2417-0.3515 | 0.3492-0.4264 |
| **P value** | 0.0221 | | 0.133 | | 0.035 | |

### [Example 20] Measurement of Serum Samples from Lung Cancer Patients

The serum samples of healthy individuals used in this Example are the same as those used in Examples 15 and 16 and Comparative Example 1. The details of the serum samples of lung cancer patients used in this Example are shown in Table 17. The serum samples of lung cancer patients were purchased from ProMedDx, LLC. It is clearly described in the documents attached to the products of the company that these samples were collected in accordance with the protocols approved by the ethics committee.

[Table 17]

**(Table 17)**

| **Sample ID** | **Age** | **Sex** | **Lung cancer stage** | **Tissue type** |
|---|---|---|---|---|
| L01 | 67 | Male | IA | Small cell lung cancer |
| L02 | 74 | Male | IIIA | Small cell lung cancer |
| L03 | 68 | Male | IIIA | Small cell lung cancer |
| L06 | 64 | Male | IIIB | Small cell lung cancer |
| L07 | 75 | Female | IIB | Small cell lung cancer |
| L08 | 69 | Male | IIA | Small cell lung cancer |
| L09 | 64 | Female | IV | Small cell lung cancer |
| L14 | 60 | Female | IB | Small cell lung cancer |
| L15 | 76 | Female | IIB | Small cell lung cancer |
| L16 | 69 | Female | IIA | Small cell lung cancer |
| L17 | 76 | Female | IIIA | Small cell lung cancer |
| L18 | 60 | Female | IIIB | Small cell lung cancer |
| nSL01 | 60 | Male | IIA | Non small cell lung cancer |
| nSL02 | 64 | Male | II | Non small cell lung cancer |

The above-described samples were measured by sandwich ELISA described in Conditions 1 to 3 of Table 7. The specific method is the same as in Example 15.

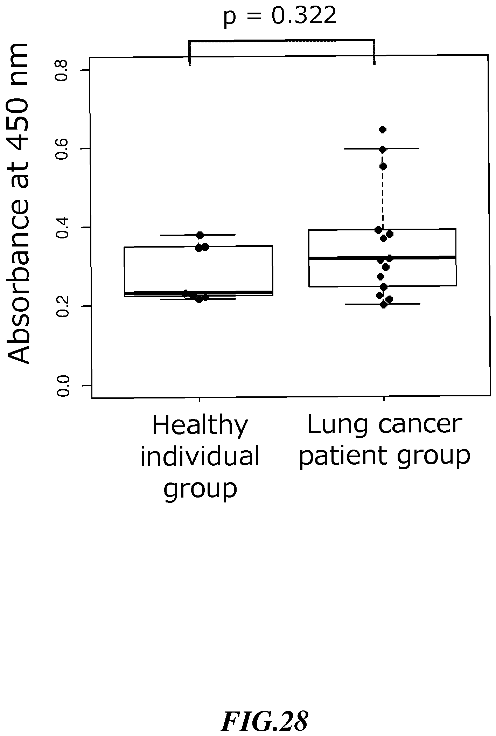

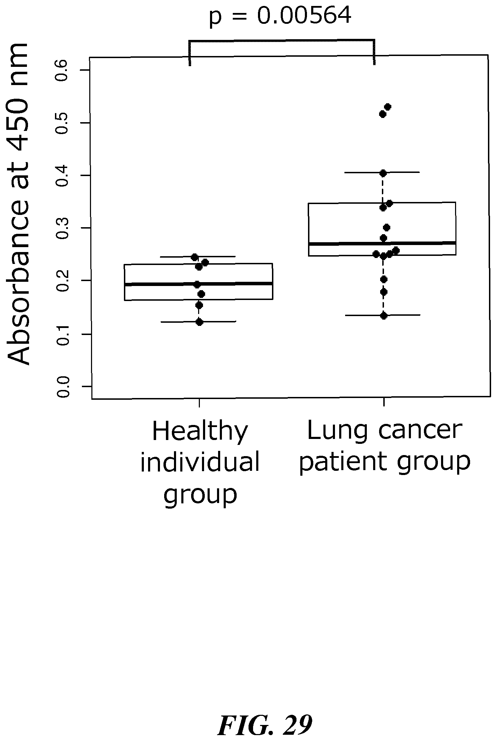

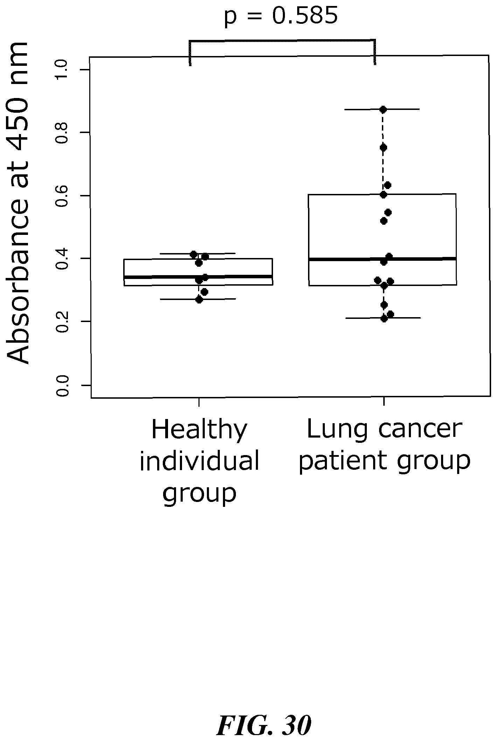

FIGS. 28 to 30 show box plots of absorbance. The minimum value, 25th percentile, median, 75th percentile, and maximum value of absorbance, and range of absorbance in the 95% confidence interval of each of the healthy individual group and the lung cancer patient group, and the p-values of the Mann-Whitney U test are shown in Table 18. In all cases of sandwich ELISA, the values in the lung cancer patient group tended to be higher than those in the healthy individual group. In particular, in the cases of using the combinations of the solid phase antibody and the biotin-labeled antibody in Example 20-2, a statistically significant difference was confirmed (p < 0.05).

[Table 18]

**(Table 18)**

| **Example** | 20-1 | | 20-2 | | 20-3 | |
|---|---|---|---|---|---|---|
| **Solid-phase antibody** | Anti-CD81 antibody | | Anti-CD9 antibody | | Anti-CD63 antibody | |
| **Biotin-labeled antibody** | Anti-AZUl antibody | | Anti-AZUl antibody | | Anti-AZU1 antibody | |
| **Group** | Healty | Lung cancer | Healty | Lung cancer | Healty | Lung cancer |
| **Minimum** | 0.2169 | 0.2009 | 0.1202 | 0.1313 | 0.2729 | 0.2079 |
| **25th Percentile** | 0.2256 | 0.2525 | 0.1639 | 0.2455 | 0.3134 | 0.3147 |
| **Median** | 0.2322 | 0.3173 | 0.1929 | 0.2672 | 0.3400 | 0.3964 |
| **75th Percentile** | 0.3499 | 0.3886 | 0.2312 | 0.3423 | 0.3981 | 0.5874 |
| **Maximum** | 0.3791 | 0.6470 | 0.2444 | 0.5287 | 0.4161 | 0.8719 |
| **95% Confidence interval** | 0.2205-0.3450 | 0.2787-0.4388 | 0.1529-0.2321 | 0.2360-0.3655 | 0.3027-0.3979 | 0.3406-0.5679 |
| **P value** | 0.322 | | 0.00564 | | 0.585 | |

### [Example 21] Measurement of Free AZU1 in Serum Samples from Healthy Individuals and Various Cancer Patients

The details of the serum samples used in this Example are shown in Table 19. All serum samples from healthy individuals were collected at the Health Service Center of the Tokyo Research Center of Tosoh Corporation with the approval of the ethics committee of the Bioscience Division of Tosoh Corporation and the consent of the sample providers. The serum samples of lung cancer, colorectal cancer, breast cancer, and stomach cancer patients were purchased from ProMedDx, LLC, and BioIVT. It is clearly described in the documents attached to the products of both companies that these samples were collected in accordance with the protocols approved by the ethics committee.

[Table 19]

**(Table 19)**

| **Example** | | 21 | | | | |
|---|---|---|---|---|---|---|
| **Group** | | Healthy | Lung cancer | Colorectal cancer | Breast cancer | Stomach cancer |
| **Number of samples** | | 28 | 17 | 15 | 11 | 17 |
| **Sex** | | | | | | |
| | **Male** | 7 | 9 | 6 | 0 | 11 |
| | **Female** | 21 | 8 | 9 | 11 | 6 |
| **Age** | | | | | | |
| | **Median** | 44.0 | 69.0 | 63.0 | 68.0 | 70.0 |
| | **Interquartile range** | 33.8-53.0 | 64.0-71.0 | 57.5-65.0 | 65.0-72.5 | 62.0-74.0 |

The concentration of free AZU1 contained in the above-described serum samples was measured by sandwich ELISA using a commercially available AZU1 ELISA kit (manufactured by Sino Biological, Product No. SEK10660). The AZU1 measured by this Example is both AZU1 present as a soluble protein and AZU1 present on cell-secreted fine particles.

The specific method is described below.
[1] A rabbit anti-human AZU1 monoclonal antibody, which was diluted with PBS to 2 µg/mL, was added at 100 µL/well to a Maxisorp 96-well microplate (manufactured by Thermo Fisher Scientific), and the mixture was allowed to stand overnight at 4°C to be immobilized.
[2] The plate was washed three times with TBS containing 0.05% Tween 20. TBS containing 2% BSA and 0.05% Tween 20 was added to the plate at 300 µL/well. The plate was then left to stand at room temperature for one hour.
[3] The plate was washed three times with TBS containing 0.05% Tween 20. A serum sample which was diluted 10-fold with TBS containing 0.1% BSA, 0.05% Tween 20, and a 0.05 mg/mL heterophilic blocking reagent (HBR1) (manufactured by Scantibodies Laboratory) or known concentrations of reference standard prepared by adding recombinant human AZU1 to the above-mentioned diluent was added to the plate at 100 µL/well. The plate was then left to stand at room temperature for two hours.
[4] The plate was washed three times with TBS containing 0.05% Tween 20. An HRP-labeled rabbit anti-human AZU1 polyclonal antibody which was diluted to 0.5 µg/mL with TBS containing 0.5% BSA and 0.05% Tween 20, was added to the plate at 100 µL/well. The plate was then left to stand at room temperature for one hour.
[5] The plate was washed three times with TBS containing 0.05% Tween 20. SureBlue Reserve TMB (manufactured by SeraCare Life Sciences) was added to the plate at 200 µL/well. The plate was then left to stand at room temperature for ten minutes.
[6] A 1 mol/L phosphoric acid aqueous solution was added at 50 µL/well to stop the reaction.
[7] The absorbance at 450 nm was measured using an absorbance plate reader.
[8] A calibration curve was prepared using recombinant AZU1 as a reference standard, and the concentration of free AZU1 in the sample was calculated.

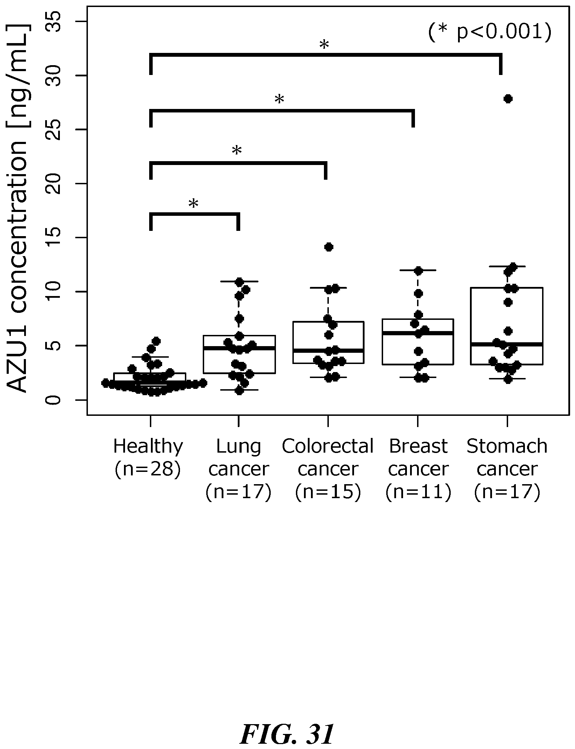

FIG. 31 shows box plots of free AZU1 concentration in serum samples. The minimum value, 25th percentile, median, 75th percentile, and maximum value of absorbance, and range of free AZU1 concentration in the 95% confidence interval of each of the healthy individual group and the groups of various cancer types, and the p-values of the Mann-Whitney U test where the healthy individual group was compared with each cancer type group are shown in Table 20. In all cancer types, the values tended to be higher than those in the healthy individual group, showing a statistically significant difference (p < 0.05) .

[Table 20]

**(Table 20)**

| **Example** | 21 | | | | |
|---|---|---|---|---|---|
| **Measurement Item** | AZU1 [ng/mL] | | | | |
| **Group** | Healthy | Lung cancer | Colorectal cancer | Breast cancer | Stomach cancer |
| **Minimum** | 0.76 | 0.88 | 2.00 | 2.02 | 1.87 |
| **25th Percentile** | 1.22 | 2.39 | 3.37 | 3.27 | 3.22 |
| **Median** | 1.54 | 4.69 | 4.48 | 6.14 | 5.03 |
| **75th Percentile** | 2.29 | 5.90 | 7.20 | 7.42 | 10.32 |
| **Maximum** | 5.41 | 10.89 | 14.21 | 11.99 | 27.92 |
| **95% Confidence interval** | 1.50-2.44 | 3.44-6.48 | 3.83-7.60 | 3.80-7.92 | 4.21-10.50 |
| **P value** | | <0.001 | <0.001 | <0.001 | <0.001 |

### INDUSTRIAL APPLICABILITY

The present invention provides a method for detecting cancer in a simple and highly accurate manner and a reagent that can be used in the method. These are significantly industrially applicable because they can be suitably used for screening various cancers, determination of therapeutic effects, and postoperative follow-up observation.

## Claims

1. A method for detecting cancer (excluding renal cell cancer), which comprises measuring a level of Azurocidin (AZU1) in a sample,
wherein it is determined that cancer is detected when a measured value exceeds a preset reference value.

2. The method according to claim 1, wherein the cancer is selected from the group consisting of stomach cancer, breast cancer, colorectal cancer, and lung cancer.

3. The method according to claim 1 or 2, wherein the level of AZU1 is measured using an antibody that specifically recognizes AZU1.

4. The method according to any one of claims 1 to 3, which comprises further detecting a second marker present on cell-secreted fine particles that are cell-secreted fine particles on which AZU1 is present in the sample,
wherein the second marker is at least one of second markers listed in Table 2.

5. The method according to claim 4, wherein the second marker contains at least one of CD81, CD63, CD9, and phosphatidylserine.

6. The method according to claim 4 or 5, wherein the second marker is detected using an antibody or receptor that specifically recognizes the second marker.

7. A reagent for use in detecting cancer (excluding renal cell cancer), which contains an antibody that specifically recognizes AZU1.

8. The reagent according to claim 7, wherein the cancer is selected from the group consisting of stomach cancer, breast cancer, colorectal cancer, and lung cancer.

9. The reagent according to claim 7 or 8, which further contains an antibody or receptor that specifically recognizes any of second markers listed in Table 2.

10. The reagent according to claim 9, wherein the second marker contains at least one of CD81, CD63, CD9, and phosphatidylserine.


SEQUENCE LISTING

<110> Japanese Foundation for Cancer Research
Osaka University
Tosoh Corporation

<120> Method and reagent for cancer detecting

<130> 2190012-0366

<150> JP2019-082912
<151> 2019-04-24

<160> 2

<170> PatentIn version 3.5

<210> 1
<211> 8
<212> PRT
<213> Homo sapiens

<400> 1

Asp Tyr Lys Asp Asp Asp Asp Lys
1               5

<210> 2
<211> 7
<212> PRT
<213> Homo sapiens

<400> 2

Cys Lys Val Leu Arg Arg His
1               5